(19) Europäisches Patentamt / European Patent Office / Office européen des brevets

(11) **EP 4 112 746 A1**

(12) **EUROPEAN PATENT APPLICATION**

(43) Date of publication:
**04.01.2023  Bulletin 2023/01**

(51) International Patent Classification (IPC):
***C12Q 1/6886*** (2018.01)

(21) Application number: **21183549.1**

(52) Cooperative Patent Classification (CPC):
**C12Q 1/6886;** C12Q 2600/106; C12Q 2600/158

(22) Date of filing: **02.07.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **STRATIFYER Molecular Pathology GmbH**
**50935 Köln (DE)**

(72) Inventors:
• **Wirtz, Markus Ralf**
**50677 Köln (DE)**

• **Brückl, Wolfgang**
**91080 Uttenreuth (DE)**
• **Brückl, Natalie**
**50937 Köln (DE)**

(74) Representative: **Michalski Hüttermann & Partner Patentanwälte mbB**
**Kaistraße 16A**
**40221 Düsseldorf (DE)**

Remarks:
The complete document including Reference Table(s) and the Sequence Listing(s) can be downloaded from the EPO website

(54) **METHOD FOR PREDICTING A CLINICAL RESPONSE TOWARDS AN IMMUNE CHECKPOINT INHIBITOR BASED ON PRETREATMENT THEREWITH**

(57)    The present invention relates to a method for predicting a clinical response in a human or animal subject suffering from or at risk of developing a neoplastic disease towards at least one given mode of treatment, said method comprising the steps of: administering to the subject at least one initial dose of an immune checkpoint inhibitor, after said initial administration, obtaining a biological sample from said subject, determining, in said sample, the expression level of at least one gene of interest selected rom the group consisting of PD1, PD-L1, CTLA4, CD3, CD4, CD68, CD163 and CD8, and predicting therapeutic success for follow up treatment with at least one immune checkpoint inhibitor

Fig. 1

EP 4 112 746 A1

**Description**

**Field of the invention**

The present application relates to the field of molecular diagnostics.

**Background**

Immune-checkpoint inhibitors (ICI) directed, *inter alia,* against programmed death 1 (PD-1), programmed death ligand 1 (PD-L1) or cytotoxic T-lymphocyte-associated protein 4 (CTLA4) have expanded the treatment options for neoplastic diseases, including advanced non small cell lung cancer (NSCLC).

In patients without druggable driver mutations ICI were first approved for palliative 2nd and 3rd line treatment of metastatic NSCLC. Up to 16% of NSCLC patients receiving an ICI as palliative 2nd or 3rd line treatment survive for more than five years. In other neoplastic diseases, similar success rates have been reported.

Nowadays, ICI either alone or as combination therapy with ICI are established in the 1st line setting. Especially, the PD-1 ICI pembrolizumab is approved in Europe as a 1st line monotherapy for patients with an immunohistochemically based tumor proportion score (TPS) for PD-L1 $\geq$ 50% after data from the registrational phase III trial KEYNOTE-024 showed superior results in comparison to a chemotherapy only control arm in terms of its primary endpoints PFS and OS.

Recently, 5-year survival data were reported with 31.9% of patients living after 1st line treatment with this ICI in contrast to 16.3% with a chemotherapy only first-line treatment (HR 0.62).

However, there is a rapid decline in the Kaplan-Meier curves treated with ICI with about one third of patients not having a long-lasting benefit < 6 months but suffering from early progression, hyperprogression or serious adverse event. These patients undergo a treatment, with all side effects involved, without receiving substantial benefit thereof.

It is hence one object of the present invention to provide reliable prognostic and predictive factors showing non-effectiveness of immune checkpoint inhibitors before the first radiological measurement of response.

It is one further object of the present invention to provide a method that better helps to predict therapeutic success of immune checkpoint inhibitors in patients suffering from a neoplastic disease.

It is one further object of the present invention to avoid side effect prone immune checkpoint inhibitor therapies for cancer patients that do not receive substantial benefit thereof.

These and further objects are met with methods and means according to the independent claims of the present invention. The dependent claims are related to specific embodiments.

**Detailed Description of the Invention**

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a", "an", and "the" include singular and/or plural referents unless the context clearly dictates otherwise. It is moreover to be understood that, in case parameter ranges are given which are delimited by numeric values, the ranges are deemed to include these limitation values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that does not necessarily mean that said feature is not meant to be disclosed with said other embodiment. The skilled person would understand that it is the gist of this application to disclose said feature also for the other embodiment, but that just for purposes of clarity and to keep the specification in a manageable volume this has not been done.

Furthermore, the content of the prior art documents referred to herein is incorporated by reference. This refers, particularly, for prior art documents that disclose standard or routine methods. In that case, the incorporation by reference has mainly the purpose to provide sufficient enabling disclosure, and avoid lengthy repetitions.

Short description of the Figures

Figure 1.     Kaplan Meier curves of the whole cohort A) PFS B) OS.
Figure 2.     Kaplan-Meier curves for PFS according to A) PD-L1 TPS score and B) to PD-L1 mRNA in whole blood.
Figure 3.     Kaplan-Meier curves of PFS according to dynamic changes: A) $\Delta$ CD3, B) $\Delta$ CD8 and C) $\Delta$ TCLA4.
Figure 4.     Kaplan-Meier curves of OS according to dynamic changes: A) $\Delta$ CD3, B) $\Delta$ CD8 and C) $\Delta$ TCLA4.
Figure 5.     Changes in relative gene expression of blood cell markers before and after immune checkpoint therapy

Table 1. Patients' demographics. NOS, not other specified; PS, performance status; TPS, tumor proportion score

Table 2. median PFS and OS data of the whole cohort with clinical and histopathological data. n.r., not reported: NOS, not other specified; PS, performance status; TPS, tumor proportional score

Table 3. median PFS and OS data of mRNA based gene expressions from the whole blood. n.r., not reported; Δ, change in expression before and after start of pembrolizumab

Table 4. Univariate and multivariate analysis of PFS in Δ gene expression. Δ, change in expression before and after start of pembrolizumab

Table 5. Univariate and multivariate analysis of OS in Δ gene expression. Δ, change in expression before and after start of pembrolizumab

According to a first aspect of the invention, a method is provided for predicting a clinical response in a human or animal subject suffering from or at risk of developing a neoplastic disease towards at least one given mode of treatment

Said method comprises the steps of:

c) administering to the subject at least one initial dose of an immune checkpoint inhibitor

d) after said initial administration, obtaining a biological sample from said subject

e) determining, in said sample, the post-treatment expression level of at least one gene of interest selected from the group consisting of PD1, PD-L1, CTLA4, CD3, CD4, CD68, CD163 and CD8, and

f) predicting therapeutic success for follow on treatment with

i) at least the immune checkpoint inhibitor of step c) or

ii) at least one other immune checkpoint inhibitor.

As used herein, the term "expression level of a gene" refers to the expression of either mRNA or protein, when such expression is measured quantitatively, based on methods as disclosed herein and/or routinely known to the skilled person.

As used herein, the term "post-treatment" relates to steps and conditions that are carried out or occur after initial treatment (e.g. after 1st or second cycle) of the immune checkpoint inhibitor.

As used herein, the term "pre-treatment" relates to steps and conditions that are carried out or occur before initiation of the immune checkpoint inhibitor therapy.

An immune checkpoint inhibitor is a form of cancer immunotherapy drug that target an immune checkpoint, i.e., a key regulator of the immune system that stimulates or inhibits its actions. Tumors can use these checkpoints to protect themselves from attacks by the immune system. Immune checkpoint therapy can block inhibitory checkpoints, restoring immune system function. Examples of immune checkpoint inhibitors will be discussed in more detail hereinbelow.

In one embodiment, said neoplastic disease is Non Small Cell Lung Cancer (NSCLC). In yet another embodiment said neoplastic disease is bladder cancer, gastric cancer, cholangiocarcinoma, breast cancer, ovarian cancer, colorectal cancer as the dynamic change of said immunological markers has to be regarded an indicator of general, systemic response which is independent of the primary cancer indication.

According to one embodiment, the method further comprises, before step c) the steps of

a) obtaining a biological sample from said subject, and

b) determining, in said sample, the pre-treatment expression level of at least one gene of interest selected from the group consisting of PD1, PD-L1, CTLA4, CD3, CD4, CD68, CD163 and CD8, and

According to one embodiment, in order to carry out step f), and after step e), it is determined whether the post-treatment expression level of the at least one gene of interest is high or low.

According to one embodiment, the post-treatment expression level of the at least one gene of interest is high

(i) in case it is higher than a predetermined treshold for that respective gene of interest, or

(ii) in case the post-treatment expression level determined in step e) is higher than or equal to the pre-treatment expression level in step b)

According to one embodiment, the post-treatment expression level of the at least one gene of interest is low

(i) in case it is lower than a predetermined treshold for that respective gene of interest, or

(ii) in case the post-treatment expression level determined in step e) is lower than the pre-treatment expression level in step b)

According to one embodiment,

- a high post-treatment expression level of at least one gene of interest is associated with good prognosis for treatment with at least one immune checkpoint inhibitor, and
- a low post-treatment expression level of at least one gene of interest is associated with poor prognosis for treatment with at least one immune checkpoint inhibitor, and

According to one embodiment, at least one gene of interest the expression of which is determined is a gene the gene product of which is a target of the immune checkpoint inhibitor of step f)(i) or f)(ii)

According to different embodiments, the biological sample is at least one selected from the group consisting of

- blood

- plasma

- serum

- tissue

- urine, and/or

- saliva

In one preferred embodiment the samples have non-invasively being drawn, or been drawn with low invasiveness. This offers the advantage to accompany patients under treatment and to guide response to sequential treatment modifications in order to improve efficacy with the method disclosed being used as an endpoint to predict outcome. The expression value determined from a particular patient before and/or after treatment can be compared with population based thresholds being taken as reference thereby alleviating the need of multiple sample determinations. However, even more preferred are intra-individual comparisons of the described biomarkers to evaluate the dynamic changes in the context of individual circumstances. By way of illustration and not by way of restriction these context dependent circumstances affecting the biomarker levels may include effects of previous anti-cancer therapies such as chemotherapies that affect the blood system and inter alias may result in immune cell deployment and/or neutropenia etc. Even in such scenarios of reduced absolute immune cell counts the intra-individual change of biomarker expression does significantly predict response and long term survival of cancer patients as exemplified below.

According to one embodiment, the biological sample taken in step a) is from the same source as the biological sample taken in step d).

According to different embodiments, said expression level(s) is/are determined by at least one of

(i) a hybridization based method, in which labeled, single stranded probes are used
(ii) a PCR based method, which method comprises a polymerase chain reaction (PCR)
(iii) a method based on the electrochemical detection of particular molecules, which method encompasses an electrode system to which molecules bind under creation of a detectable signal,
(iv) an array based method, which comprises the use of a m microarray and/or biochip, and/or
(v) an immunological method, in which one or more target-specific protein binders are used.

The term "a PCR based method" as used herein refers to methods comprising a polymerase chain reaction (PCR). This is an approach for exponentially amplifying nucleic acids, like DNA or RNA, via enzymatic replication, without using a living organism. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR). Moreover, PCR-based methods comprise e.g. real time PCR, and, particularly suited for the analysis of expression levels, kinetic or quantitative PCR (qPCR).

The term "Quantitative real-time PCR" (qPCR)" refers to any type of a PCR method which allows the quantification of the template in a sample. Quantitative real-time PCR comprise different techniques of performance or product detection as for example the TaqMan technique or the LightCycler technique. The TaqMan technique, for examples, uses a dual-labelled fluorogenic probe. The TaqMan real-time PCR measures accumulation of a product via the fluorophore during the exponential stages of the PCR, rather than at the end point as in conventional PCR. The exponential increase of the product is used to determine the threshold cycle, CT, i.e. the number of PCR cycles at which a significant exponential increase in fluorescence is detected, and which is directly correlated with the number of copies of DNA template present in the reaction. The set up of the reaction is very similar to a conventional PCR, but is carried out in a real-time thermal cycler that allows measurement of fluorescent molecules in the PCR tubes. Different from regular PCR, in TaqMan real-time PCR a probe is added to the reaction, i.e., a single-stranded oligonucleotide complementary to a segment of 20-60 nucleotides within the DNA template and located between the two primers. A fluorescent reporter or fluorophore (e.g., 6-carboxyfluorescein, acronym: FAM, or tetrachlorofluorescin, acronym: TET) and quencher (e.g., tetramethylrhodamine, acronym: TAMRA, of dihydrocyclopyrroloindole tripeptide "minor groove binder", acronym: MGB) are covalently attached to the 5' and 3' ends of the probe, respectively [2]. The close proximity between fluorophore and quencher attached to the probe inhibits fluorescence from the fluorophore. During PCR, as DNA synthesis commences, the 5' to 3' exonuclease activity of the Taq polymerase degrades that proportion of the probe that has annealed to the template (Hence its name: Taq polymerase+PacMan). Degradation of the probe releases the fluorophore from it and breaks the close proximity to the quencher, thus relieving the quenching effect and allowing fluorescence of the fluorophore. Hence, fluorescence detected in the realtime PCR thermal cycler is directly proportional to the fluorophore released and the amount of DNA template present in the PCR.

A "microarray" herein also refers to a "biochip" or "biological chip". an array of regions having a density of discrete regions of at least about 100/cm$^2$, and preferably at least about 1000/cm$^2$. The regions in a microarray have typical dimensions, e.g., diameters, in the range of between about 10-250 $\mu$m, and are separated from other regions in the array by about the same distance.

The term "hybridization-based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. These approaches are also known as "array based methods". Yet another hybridization based method is PCR, which is described above. When it comes to the determination of expression levels, hybridization based methods may for example be used to determine the amount of mRNA for a given gene.

The term "method based on the electrochemical detection of molecules" relates to methods which make use of an electrode system to which molecules, particularly biomolecules like proteins, nucleic acids, antigens, antibodies and the like, bind under creation of a detectable signal. Such methods are for example disclosed in WO0242759, WO0241992 and WO02097413 filed by the applicant of the present invention, the content of which is incorporated by reference herein. These detectors comprise a substrate with a planar surface which is formed, for example, by the crystallographic surface of a silicon chip, and electrical detectors which may adopt, for example, the shape of interdigital electrodes or a two dimensional electrode array. These electrodes carry probe molecules, e.g. nucleic acid probes, capable of binding specifically to target molecules, e.g. target nucleic acid molecules. The probe molecules are for example immobilized by a Thiol-Gold-binding. For this purpose, the probe is modified at its 5'- or 3'-end with a thiol group which binds to the electrode comprising a gold surface. These target nucleic acid molecules may carry, for example, an enzyme label, like horseradish peroxidise (HRP) or alkaline phosphatase. After the target molecules have bound to the probes, a substrate is then added (e.g., $\alpha$-naphthyl phosphate or 3,3'5,5'-tetramethylbenzidine which is converted by said enzyme, particularly in a redox-reaction. The product of said reaction, or a current generated in said reaction due to an exchange of electrons, can then be detected with help of the electrical detector in a site specific manner.

The term "immunological method" refers to methods in which one or more target-specific protein binders are used. Such methods include Western Blot (WB), Immunohistochemistry (IHC), immunofluorescence (**IF**), Immunocytochemistry (**ICC**) and ELISA, all of which are routine methods. Such protein binders that are, inter alia, suitable for being used in the above methods, are e.g. poly- or monoclonal antibodies that bind to any of PD1, PD-L1, CTLA4, CD3 or CD8, or to altered variants thereof. Such antibodies can be generated by the skilled person with routine methods (immunization/hybridoma), and can also be obtained from the usual suppliers. The following table shows just a non-limiting list of examples:

| Type | Catalog number/Clone ID | Supplier |
|---|---|---|
| Anti PD1 antibody | ET1606-41) | Huabio |
| Anti PD-L1 antibody | ab205921 | Abcam |

(continued)

| Type | Catalog number/Clone ID | Supplier |
|---|---|---|
| | 22C3 | DAKO |
| | 28-8 | DAKO |
| | SP142 | Roche |
| | SP263 | Roche |
| Anti CTLA4 antibody | MAB325-SP | R&D systems |
| Anti CD8 antibody | BLR044F | Bethyl |
| Anti CD3 antibody (any of CD3γ, CD3δ or CD3ε) | 12F6 (Rabbit IgGκ) | Enzo |
| Anti CD4 antibody | M-T466 | Miltenyi |
| Anti CD68 antibody | sc-20060 | Santa Cruz Biotechnology |
| Anti CD163 antibody | 10D6 | Thermo Fisher |
| Anti CD8 antibody | BLR044F | Bethyl |

According to one embodiment. said expression level(s) is/are determined by real time polymerase chain reaction (RT-PCR or qPCR) of at least one of the genes of interest.

In RT-PCR or qPCR, the amplification of the targeted DNA molecule is monitored during the PCR, i.e. in real-time, and not at its end, as in conventional PCR. Real-time PCR can be used quantitatively (quantitative real-time PCR), and semi-quantitatively, i.e. above/below a certain amount of DNA molecules (semi quantitative real-time PCR).

Two common methods for the detection of PCR products in real-time PCR are: (1) non-specific fluorescent dyes that intercalate with any double-stranded DNA, and (2) sequence-specific DNA probes consisting of oligonucleotides that are labelled with a fluorescent reporter which permits detection only after hybridization of the probe with its complementary sequence.

One measure for the expression level of a given gene is Ct ("cycle threshold"). Ct is defined as the number of cycles required for the fluorescent signal to cross the threshold (i.e., to exceed background level). Ct levels are inversely proportional to the amount of target mRNA in the sample, i.e., the lower the Ct level the greater the amount of target mRNA in the sample, i.e., the higher the respective gene expression level is.

According to one or more embodiments of the invention, the method is characterized in that the one or more expression level(s) determined in step a) are normalized with one or more expression level(s) of one or more reference genes before step b) to obtain one or more normalized expression level(s)

Reference genes in PCR are discussed in Kozera and Rapacz (2013), the content of which is incorporated herein by reference.

In order to normalize the expression level of a given gene, a comparison to a reference gene is preferably made. In one embodiment, the normalized gene expression of any one of the genes of interest is calculated by the following formula:

$$40 - ((Ct\ target\ gen) - (\ Ct\ housekeeper))$$

also called "ΔCT" or "40-DCT" herein.

Hence, in one embodiment, in which the post-treatment expression level of the at least one gene of interest is considered to be high in case the expression level determined in step e) is higher than or equal to the expression level in step b), the 40-DCT is considered. In case the 40-DCT determined post-treatment in step e) (i.e., after the initial administration of the immune checkpoint inhibitor) is higher than or equal to the 40-DCT determined pre-treatment in step b), said expression level is considered to be high.

In case the 40-DCT determined post-treatment in step e) (i.e., after the initial administration of the immune checkpoint inhibitor) is lower than the 40-DCT determined pre-treatment in step b), said expression level is considered to be low.

According to one embodiment, the one or more post-treatment expression level(s) determined in step e) are normalized with one or more expression level(s) of one or more reference genes to obtain one or more normalized expression level(s).

According to one embodiment, said one or more reference gene(s) is at least one housekeeping gene.

According to one embodiment, the at least one housekeeping gene is selected from the group consisting of CALM2, B2M and/or RPL37A.

According to one embodiment, the sample is treated with germanium beads or silica beads, or magnetic beads coated with germanium or silica, and a chaotropic salt, for purification of the nucleic acids contained in said sample prior to the determination in step e) and/or b)).

According to different embodiments, the immune checkpoint inhibitor is at least one selected from the group consisting of

- PD-1 inhibitor

- PD-L1 inhibitor

- CTLA-4 inhibitor

- LAG 3, inhibitor

- TIM3 inhibitor, and/or

- OX40 inhibitor

According to different embodiments, the immune checkpoint inhibitor is at least one selected from the group consisting of an

- antibody,
- modified antibody format,
- antibody derivative or fragment retaining target binding properties
- antibody-based binding protein,
- oligopeptide binder and/or
- an antibody mimetic.

"Antibodies", also synonymously called "immunoglobulins" (Ig), are generally comprising four polypeptide chains, two heavy (H) chains and two light (L) chains, and are therefore multimeric proteins, or an equivalent Ig homologue thereof (e.g., a camelid nanobody, which comprises only a heavy chain, single domain antibodies (dAbs) which can be either be derived from a heavy or light chain); including full length functional mutants, variants, or derivatives thereof (including, but not limited to, murine, chimeric, humanized and fully human antibodies, which retain the essential epitope binding features of an Ig molecule, and including dual specific, bispecific, multispecific, and dual variable domain immunoglobulins; Immunoglobulin molecules can be of any class (e.g., IgG, IgE, IgM, IgD, IgA, and IgY), or subclass (e.g., IgG1, IgG2, IgG3, IgG4, IgA1, and IgA2) and allotype.

An "antibody-based binding protein", as used herein, may represent any protein that contains at least one antibody-derived $V_H$, $V_L$, or $C_H$ immunoglobulin domain in the context of other non-immunoglobulin, or non-antibody derived components. Such antibody-based proteins include, but are not limited to (i) $F_c$-fusion proteins of binding proteins, including receptors or receptor components with all or parts of the immunoglobulin $C_H$ domains, (ii) binding proteins, in which $V_H$ and or $V_L$ domains are coupled to alternative molecular scaffolds, or (iii) molecules, in which immunoglobulin $V_H$, and/or $V_L$, and/or $C_H$ domains are combined and/or assembled in a fashion not normally found in naturally occurring antibodies or antibody fragments.

An "antibody derivative or fragment", as used herein, relates to a molecule comprising at least one polypeptide chain derived from an antibody that is not full length, including, but not limited to (i) a Fab fragment, which is a monovalent fragment consisting of the variable light ($V_L$), variable heavy ($V_H$), constant light ($C_L$) and constant heavy 1 ($C_H$1) domains; (ii) a F(ab')2 fragment, which is a bivalent fragment comprising two Fab fragments linked by a disulfide bridge at the hinge region; (iii) a heavy chain portion of a $F_{ab}$ ($F_d$) fragment, which consists of the $V_H$ and $C_H$1 domains; (iv) a variable fragment ($F_v$) fragment, which consists of the $V_L$ and $V_H$ domains of a single arm of an antibody, (v) a domain antibody (dAb) fragment, which comprises a single variable domain; (vi) an isolated complementarity determining region (CDR); (vii) a single chain $F_v$ Fragment (sc$F_v$); (viii) a diabody, which is a bivalent, bispecific antibody in which $V_H$ and $V_L$ domains are expressed on a single polypeptide chain, but using a linker that is too short to allow for pairing between the two domains on the same chain, thereby forcing the domains to pair with the complementarity domains of another chain and creating two antigen binding sites; and (ix) a linear antibody, which comprises a pair of tandem $F_v$ segments ($V_H$-$C_H$1-$V_H$-$C_H$1) which, together with complementarity light chain polypeptides, form a pair of antigen binding regions; and (x) other non-full length portions of immunoglobulin heavy and/or light chains, or mutants, variants, or derivatives thereof, alone or in any combination. In any case, said derivative or fragment retains target binding properties

The term "modified antibody format", as used herein, encompasses antibody-drug-conjugates, Polyalkylene oxide-modified scFv, Monobodies, Diabodies, Camelid Antibodies, Domain Antibodies, bi- or trispecific antibodies, IgA, or two IgG structures joined by a J chain and a secretory component, shark antibodies, new world primate framework + non-new world primate CDR, IgG4 antibodies with hinge region removed, IgG with two additional binding sites engineered into the CH3 domains, antibodies with altered Fc region to enhance affinity for Fc gamma receptors, dimerised constructs comprising CH3+VL+VH, and the like.

The term "antibody mimetic", as used herein, refers to proteins not belonging to the immunoglobulin family, and even non-proteins such as aptamers, or synthetic polymers. Some types have an antibody-like beta-sheet structure. Potential advantages of "antibody mimetics" or "alternative scaffolds" over antibodies are better solubility, higher tissue penetration, higher stability towards heat and enzymes, and comparatively low production costs.

Some antibody mimetics can be provided in large libraries, which offer specific binding candidates against every conceivable target. Just like with antibodies, target specific antibody mimetics can be developed by use of High Throughput Screening (HTS) technologies as well as with established display technologies, just like phage display, bacterial display, yeast or mammalian display. Currently developed antibody mimetics encompass, for example, ankyrin repeat proteins (called DARPins), C-type lectins, A-domain proteins of S. aureus, transferrins, lipocalins, 10th type III domains of fibronectin, Kunitz domain protease inhibitors, ubiquitin derived binders (called affilins), gamma crystallin derived binders, cysteine knots or knottins, thioredoxin A scaffold based binders, SH-3 domains, stradobodies, "A domains" of membrane receptors stabilised by disulfide bonds and Ca2+, CTLA4-based compounds, Fyn SH3, and aptamers (peptide molecules that bind to a specific target molecules).

According to different embodiments, the immune checkpoint inhibitor is at least one selected from the group as set forth in table 6.

**Table 6: Immune checkpoint inhibitors**

| Target | Drug name | Drug type | mAb isotype |
|---|---|---|---|
| CTLA-4 | Ipilimumab | Human mAb | IgGlk |
| | Tremelimumab | Human mAb | IgG2k |
| | AGEN-1884 | Human mAb | IgG1 |
| PD-1 | Pembrolizumab | Humanized mAb | IgG4k |
| | Nivolumab | Human mAb | IgG4k |
| | PDR001 | Humanized mAb | IgG4 |
| | SHR1210 | Humanized mAb | IgG4k |
| | Cemiplimab | Human mAb | *IgG4* |
| | REGN2810 | *Human mAb* | IgG4 |
| | Pidilizumab | Humanized mAb | IgGlk |
| | AMP 514 | Humanized mAb | IgG4k |
| | BGB A317 | Humanized mAb | IgG4 |
| | PF-06801591 | mAb | - |
| | AMP224 | Fusion protein of PD-L2 and Fc domain of human IgG | NA |
| PD-L1 | Atezolizumab | Humanized mAb | IgGlk |
| | Durvalumab | Human mAb | IgGlk |
| | Avelumab | Human mAb | IgGlk |
| | CK-301 | Checkpoint Therapeutics | fully human antibody |
| | BMS 936559 | Human mAb | IgG4 |
| 7-H3 | MGA-271 | Humanized mAb | IgG1 |
| | MGD-009 | B7-H3 x CD3 DART protein | NA |

(continued)

| Target | Drug name | Drug type | mAb isotype |
|--------|-----------|-----------|-------------|
| LAG-3 | IMP-321 | LAG-3 and human IgG1 fusion protein | NA |
|  | BMS-986016 | mAb | - |
|  | LAG-525 | Humanized mAb | IgG4 |
| TIM-3 | TSR-022 | Humanized mAb | IgG4 |
|  | MBG-453 | mAb | - |
| VISTA | CA-170 | Small-molecule antagonist | NA |
| GITR | TRX-518 | Humanized mAb | IgG1 |
|  | INCAGN01876 | Human mAb | IgG1 |
|  | GWN-323 | Human mAb | IgG1 |
|  | MEDI1873 | Human mAb | IgG1 |
|  | MK-4166 | Human mAb | IgG1 |
|  | MK-1248 | mAb | - |
|  | BMS986156 | mAb | - |
| CD27 | Varlilumab | Human | IgGlk |
| CD70 | SGN-CD70A | mAb | - |
| CD40 | ISF35 | Adenovirus vector | NA |
|  | RO70097890 | mAb | - |
| OX40 | MEDI-6469 | mAb | Murine IgG1 |
|  | MOXR-0916 | Humanized mAb | IgG1 |
|  | PF-04518600 | Human mAb | IgG2 |
|  | MEDI-0562 | Humanized mAb | IgG1 |
| 4-1BB | Urelumab | Human mAb | IgG4k |
|  | Utomilumab | Human mAb | IgG2 |
| **DART, Dual-Affinity Re-Targeting; mAb, monoclonal antibody; NA, not applicable.** | | | |

In one embodiment, an anti PD1 antibody is preferred. In one embodiment, Pembrolizumab is preferred. In another embodiment, Nivolumab is preferred.

In one embodiment, an anti PD-L1 antibody is preferred. In one embodiment, Atezolizumab is preferred. In another embodiment, Durvalumab is preferred. In another embodiment, Avelumab is preferred.

According to another aspect of the invention, an oligonucleotide is provided comprising at least one nucleotide sequence which is capable of hybridizing to

a) a nucleic acid molecule that encodes for any one of PD1, PD-L1, CTLA4, CD3, CD4, CD68, CD163 and CD8, or,
b) an mRNA that encodes for any one of PD1, PD-L1, CTLA4, CD3, CD4, CD68, CD163 and CD8.

The oligonucleotide is selected from the group consisting of

- an amplification primer (forward and/or reverse)
- a labelled probe, and/or
- a substrate bound probe

The oligonucleotide is provided for the manufacture of a kit for carrying out a method of the above disclosure.

"Primer" and "probes", within the meaning of the invention, shall have the ordinary meaning of this term which is well known to the person skilled in the art of molecular biology. In a preferred embodiment of the invention "primers" and "probes", shall be understood as being polynucleotide molecules having a sequence identical, complementary, homologous, or homologous to the complement of regions of a target polynucleotide which is to be detected or quantified. In yet another embodiment nucleotide analogues and /or morpholinos are also comprised for usage as primers and/or probes.

The following table shows respective genomic sequences and selected mRNA sequences of the human variants of PD1, PD-L1, CTLA4, CD3, CD4, CD68, CD163 and CD8.

| Type | Gnomic sequence (entrez gene ID) | mRNA sequence (NCBI reference) |
|---|---|---|
| PD1 | 5133 | NM_005018.2 |
| PD-L1 | 29126 | NM_001314029 |
| CTLA4 | 1493 | NM_001037631 |
| CD3$\gamma$ | 917 | NM_000073 |
| CD3$\delta$ | 915 | NM_000732 |
| CD3$\varepsilon$ | 916 | NM_000733 |
| CD3z | | NM_000734 |
| CD8 | 926 | NM_172099 |
| CD4 | 920 | NM_000616 |
| CD68 | 968 | NM_001251 |
| CD163 | 9332 | NM_004244 |

Based on the teaching of the present invention, and the sequence information disclosed herein and in the public databases showing the genomic and mRNA sequences of PD1, PD-L1, CTLA4, CD3, CD4, CD68, CD163 and CD8, the skilled person can find likewise suitable alterative primers and probes without any inventive activity. Therefore, such alternatives shall also be encompassed by the scope of this application.

According to another aspect of the invention, a kit comprising at least one oligonucleotide set forth in the above disclosure is provided.

According to one embodiment, the kit comprises a set of forward/reverse primers capable of hybridizing to a nucleic acid molecule that encodes for at least one of PD1, PD-L1, CTLA4, CD3, CD4, CD68, CD163 and CD8.

According to one embodiment, said kit comprises at least one primer/probe set comprising a forward primer and a reverse primer, and optionally a probe, as set forth in table 7.

According to one embodiment, said comprises a labelled probe that is labelled with one or more fluorescent molecules, luminescent molecules, radioactive molecules, enzymatic molecules and/or quenching molecules.

One typical type of probe that ca be used in the context of the present invention is a so-called TaqMan probe. TaqMan probes consist of a fluorophore covalently attached to the 5'-end of the oligonucleotide probe and a quencher at the 3'-end. Several different fluorophores (e.g. 6-carboxyfluorescein, acronym: FAM, or tetrachlorofluorescein, acronym: TET) and quenchers (e.g. tetramethylrhodamine, acronym: TAMRA) are available. The quencher molecule quenches the fluorescence emitted by the fluorophore when excited by the cycler's light source via Forster resonance energy transfer (FRET). As long as the fluorophore and the quencher are in proximity, quenching inhibits any fluorescence signals. TaqMan probes are designed such that they anneal within a DNA region amplified by a specific set of primers. (Unlike the diagram, the probe binds to single stranded DNA.) TaqMan probes can be conjugated to a minor groove binder (MGB) moiety, dihydrocyclopyrroloindole tripeptide (DPI3), in order to increase its binding affinity to the target sequence; MGB-conjugated probes have a higher melting temperature (Tm) due to increased stabilisation of van dar Waals forces. As the Taq polymerase extends the primer and synthesizes the nascent strand (again, on a single-strand template, but in the direction opposite to that shown in the diagram, i.e. from 3' to 5' of the complementary strand), the 5' to 3' exonuclease activity of the Taq polymerase degrades the probe that has annealed to the template. Degradation of the probe releases the fluorophore from it and breaks the close proximity to the quencher, thus relieving the quenching effect and allowing fluorescence of the fluorophore. Hence, fluorescence detected in the quantitative PCR thermal cycler is directly proportional to the fluorophore released and the amount of DNA template present in the PCR.

According to another aspect of the invention, an immune checkpoint inhibitor is provided for use in the treatment of a human or animal subject

- being diagnosed for,

- suffering from, or
- being at risk of developing

a neoplastic disease,

wherein said subject or neoplastic disease has a high expression level in a given sample of at least one gene of interest selected from the group consisting of PD1, PD-L1, CTLA4, CD3 and CD 8, as determined after administration to said subject of at least one initial dose of an immune checkpoint inhibitor.

According to another aspect of the invention, a method of treating a human or animal subject

- being diagnosed for,
- suffering from, or
- being at risk of developing

a neoplastic disease is provided, which method comprising administering, in one or more therapeutically effective doses, an immune checkpoint inhibitor, wherein said subject or neoplastic disease has a high expression level in a given sample of at least one gene of interest selected from the group consisting of PD1, PD-L1, CTLA4, CD3 and CD 8, as determined after administration to said subject of at least one initial dose of an immune checkpoint inhibitor. The inventors have shown that these patients receive particular benefit from immune checkpoint inhibitor therapy.

On the contrary, respective patients, who, after administration to of at least one initial dose of an immune checkpoint inhibitor, show low expression levels in a given sample of at least one gene of interest selected from the group consisting of PD1, PD-L1, CTLA4, CD3 and CD 8, will likely receive less benefit from immune checkpoint inhibitor therapy. It will then have to be assessed whether the relative small benefit justifies the side effects that coincide with immune checkpoint inhibitor therapy, or whether these patients should better

a) subjected to one or more different types of cancer therapy, like e.g. chemotherapy, therapy with a tyrosine kinase inhibitor or therapy with an anti angiogenetic drug, or
b) receive, in addition to immune checkpoint inhibitor therapy, one or more further types of cancer therapy, like e.g. chemotherapy, therapy with a tyrosine kinase inhibitor or therapy with an anti angiogenetic drug.

Further, if the method of the present invention indicates no benefit and/or predicts progress despite ongoing ICI treatment, this shall e.g. enable early adaption of treatment by dosis intensification, switch from monotherapy to combination therapy by adding a chemotherapeutic agent or adding a second ICI od increasing immune response by additional immunoactive factors such as hormones, antihormones or more targeted immune stimuli such as interleukin-containing agents.

As regards the methodologies of determining said post treatment expression level of the at least one gene of interest, reference is made to the description herein, to avoid unnecessary repetitions. This involves, in preferred embodiments, the determination of the pre-treatment expression level and comparison thereof with the post treatment expression level, as well as the preferred immune checkpoint inhibitors disclosed herein, as well as the detection methods discussed herein.

Preferably, the immune checkpoint inhibitor that is subject of the treatment of the neoplastic disease is the same as the one used in the administration of the initial dose, i.e., prior to the determination of the post treatment expression level of the gene of interest.

With regard to embodiments of the immune checkpoint inhibitor that is subject of the treatment

In one embodiment, said neoplastic disease is Non Small Cell Lung Cancer (NSCLC). In yet another embodiment said neoplastic disease is bladder cancer, gastric cancer, cholangiocarcinoma, breast cancer, ovarian cancer, colorectal cancer.

**Examples**

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

All amino acid sequences disclosed herein are shown from N-terminus to C-terminus; all nucleic acid sequences disclosed herein are shown 5'->3'.

**Materials and Methods**

RNA was extracted from whole blood samples according to general instruction for use of the commercially available bead-based extraction method (Xtrakt kit; Stratifyer Molecular Pathology GmbH, Cologne, Germany). In brief, 100 $\mu$l blood or plasma were put into a 1,5 ml microcentrifuge tube and 100 $\mu$l red cell lysis buffer was added to incubate for 15 minutes at 95°C with shaking at 100 rpm in a thrmomixer. Thereafter, the lysate was treated with proteinase K for 15 minutes at 65°C. Subsequently bindng buffer and magnetic beads were added and incubated for 15 minutes at room temperature with shaking at 1200 rpm. Supernatant was discarded and the beads were washed by three cycles of adding wash buffer, aspiration, magnetization and discarding the supernatant. Finally nucleic acids were luted by adding 100 $\mu$l elution buffer and incubation at 95°C for 15 minutes while shaking at 1000 rpm. Therefater the beads were magnetized and the supernatant was DNAse I digested to receive DNAse free total RNA from the specimen. RNA eluates were then stored at -80 °C until use. The mRNA levels of CD3, CD, PD1, PD-L1 and the reference genes Calmodulin2 (CALM2) and Beta-2 microglobulin (B2M) were determined by a one-step RT-qPCR using the SuperScript III RT-qPCR system (Invitrogen, Waltham, MA, USA) and gene specific primer-probe combinations (Stratifyer). Each patient sample or control was analyzed in duplicate in an Light Cycler LC480 Instrument II (Roche Diagnostics, Rotkreuz, Switzerland) according to the manufacturers' instructions with 30 min at 50° C, 2 min at 95° C followed by 40 cycles of 15 sec at 95° C and 30 sec at 60° C. according to MammaTyper® instructions for use 140603-90020-EU Rev 2.0.. Gene expression was quantified with a modification of the method by Schmittgen and Livak by calculating 40-$\Delta$Ct, whereas $\Delta$Ct was calculated as the difference in Ct between the test gene and the mean of the reference genes. Gene expression levels were calculated as described before.: In short cycle quantification threshold (Cq) values of maker genes (MG) for each sample (S) were estimated as the median of the triplicate measurements. To correct for inter-run variations Cq values were normalized against the mean expression of the REF genes and set off against a calibrator (PC) ($\Delta\Delta$Cq method). By subtracting $\Delta\Delta$Cq from the total number of cycles (40) it was ensured that normalized gene expression is proportional to the corresponding mRNA expression levels. This method facilitates interpretation of data and clinicopathological correlations. The various calculation steps are summarized in the following formula:

$$40\text{-}\Delta\Delta Cq(MG)S = 40\text{-}((Cq[MG]S - meanCq[REF]S) - (Cq[MG]pc - meanCq[REF]pc)).$$

**Design of the study**

This retrospective analysis studies prognostic markers for effectivity under a palliative 1st line treatment with the ICI pembrolizumab in patients with metastatic NSCLC. Peripheral blood probes were taken prospectively before and during treatment with pembrolizumab. Patients had to have a histologically proven NSCLC UICC stage IV according to the 8th edition of the UICC TNM classification. Patients with a history of adjuvant chemotherapy before 1st line palliative therapy could be included, if there was an interval of more than 12 months between the adjuvant treatment and the first systemic treatment for stage IV disease. Patients with a history of palliative radiation therapy could also be enrolled into the study. NSCLC with a non-squamous histology were tested for EGFR mutations and ALK translocations and in case of a positive result were excluded. Patients had to have a PD-L1 TPS score from the tumor biopsy of $\geq$50% to qualify for 1st line pembrolizumab according to the approval status of pembrolizumab in Germany.

Treatment was performed at a high-volume German center (Nuremberg) with specialization in thoracic oncology and strong experience in chemo- and immune-oncology therapy. Patients were treated with pembrolizumab between February 2017 and October 2020, which allowed for a follow-up of at least six months prior to the data cut-off of April 30th, 2021.

Data were recorded in a standardized manner with the following data being collected for each patient: age, sex, tumor stage, histology, performance status according to ECOG (ECOG-PS), PD-L1 tumor proportional score (TPS) determined by immunohistochemistry (marker ZR3), mutational status (for non-squamous tumors) determined by next-generation sequencing (NGS), date of NSCLC diagnosis, survival status at time point of documentation (alive or deceased) and date of last contact / death. For ICI treatment the following details of therapy were documented: date of start, number of treatment cycles, best response, date of progression and reason for treatment stop.

ECOG is typically a very strong prognostic factor for survival. It is often used as exclusion criterion in trials. Ultimately, study data are based only on relatively good general condition (ECOGo, ECOG1). In reality, however, all patients are treated, especially those who are worse off. In this respect, one must ensure that the predictive marker is not determined by the ECOG and that there is a bias.

PDL1 IHC status (>50%) is a prerequisite for treatment with pembrolizumab. Efficacy at lower PD-L1 status has not been demonstrated in NSCLC. In other indications, a (weak) efficacy could not be proven even at low PD-L1, so that all are treated. It may however be used as a basic indicator.

This study was approved by the Ethics committee of the Friedrich-Alexander-University Erlangen-Nuremberg, Germany (numbers: 56_16B and 62_17B). Written informed consent for blood taking, collecting and analysing for research purpose was obtained from every patient.

**RNA isolation and quantitative reverse transcription-polymerase chain reaction (RT-qPCR assessment)**

RNA was extracted from plasma samples and processed according to a commercially available bead-based extraction method (Xtract kit; STRATIFYER Molecular Pathology GmbH, Cologne, Germany).

RNA was eluted with 100 $\mu$L of elution buffer. DNA was digested, and RNA eluates were then stored at -80 °C until use. The mRNA levels of CD3, CD, PD1, PD-L1 and the reference genes Calmodulin2 (CALM2) and Beta-2 microglobulin (B2 M) were determined by a one-step qRT-PCR using the SuperScript III RT-qPCR system (Invitrogen, Waltham, MA, USA) and gene specific primer-probe combinations (Stratifyer). Each patient sample or control was analyzed in duplicate in an Light Cycler LC480 Instrument II (Roche Diagnostics, Rotkreuz, Switzerland) according to the manufacturers' instructions. Gene expression was quantified with a modification of the method by Schmittgen and Livak by calculating 40-$\Delta$Ct, whereas $\Delta$Ct was calculated as the difference in Ct between the test gene and the mean of the reference genes.

**Abbreviations**

CD: cluster of differentiation; CI: Confidence interval; CR: Complete response; CT: Computer tomography; CTLA4: cytotoxic T-lymphocyte-associated protein 4; CTx: chemotherapy; CTx+ICI: combination of chemotherapy and immune-checkpoint inhibitor; DCR: Disease Control Rate; DOR: Duration of Response; ECOG PS: Eastern Cooperative Oncology Group performance status; HR: Hazard ratio; ICI: immune-checkpoint inhibitor; LB: liquid biopsy; mRNA: messenger ribonucleotide acid; n.r.: not reached; NSCLC: Non-small cell lung cancer; ORR: Objective Response Rate; OS: Overall Survival; RECIST: Response Evaluation Criteria in Solid Tumors version 1.1.8; PD: Progressive disease; PD1: Programmed cell Death protein 1; PD-L1: Programmed cell Death-Ligand 1; PFS: Progression-free Survival; PR: Partial response; SD: Stable disease; TTP: time to treatment progression; UICC: Union for International Cancer Control.

**Results**

**Patient population**

After excluding 7 patients treated with pembrolizumab 1st line at our center (4 patients with insufficient blood plasma probes for and after start of ICItreatment) 3 patients with insufficient follow-up data) 45 patients met the inclusion criteria. The baseline demographic data and tumor characteristics are listed in Table 1. The median age was 68 years (range 49-82), and 57.8% of the patients were male. More than half of the patients had an ECOG-PS (Eastern Cooperative Oncology Group performance status) of 0 (58.1%). PD-L1 data were available for all patients.

A TPS score of ≥50% was necessary to be treated by pembrolizumab 1st line. Of those 48.9% and 51.1% presented with a TPS score of ≥50 and <75% and ≥75%, respectively.

**Outcome of the whole cohort**

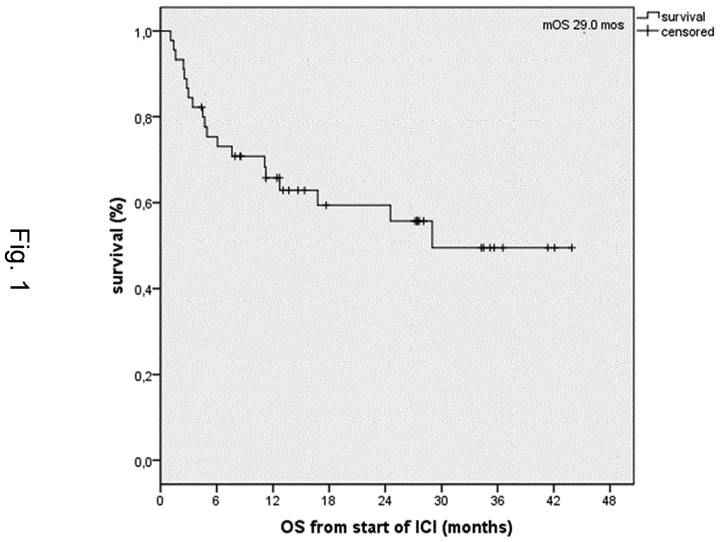

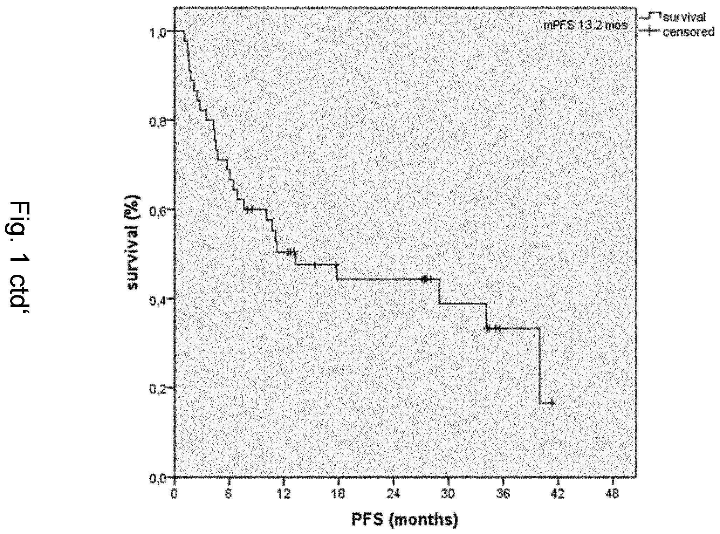

After a median follow-up of 27.4 months, a response to 1st line therapy was observed in 66.7% of patients with a median duration of response (DOR) of 34.1 months, (95%CI: 20.6-47.6 months). After a median number of 11 cycles of pembrolizumab (range 2-38) the median PFS was 13.2 months, (95%CI: 3.4-23.1 months) with 6-, 12- and 24- months PFS rates of 66.7%, 50.4% and 44.4%, respectively. 6 patients (13.3%) were still on treatment. From the start of ICI the median OS was 29 months (95%CI: not reached) with 12- and 24- months survival rates of 65.7 and 59.4%, respectively (Figure 1). In addition, clinical subgroups were tested for prognostic value in terms of PFS or OS. Beside histology (adeno-ca versus squamous cell carcinoma or NOS) which revealed a significant difference in PFS (p=0.033) and a trend for OS (p=0.061) none of the other parameters were of prognostic relevance (Table 2). While histology was a significant parameter in the univariate cox-regression analysis it did not maintain significant in the multivariate analysis (data not shown).

**Blood -based gene expression before start of ICI treatment and survival**

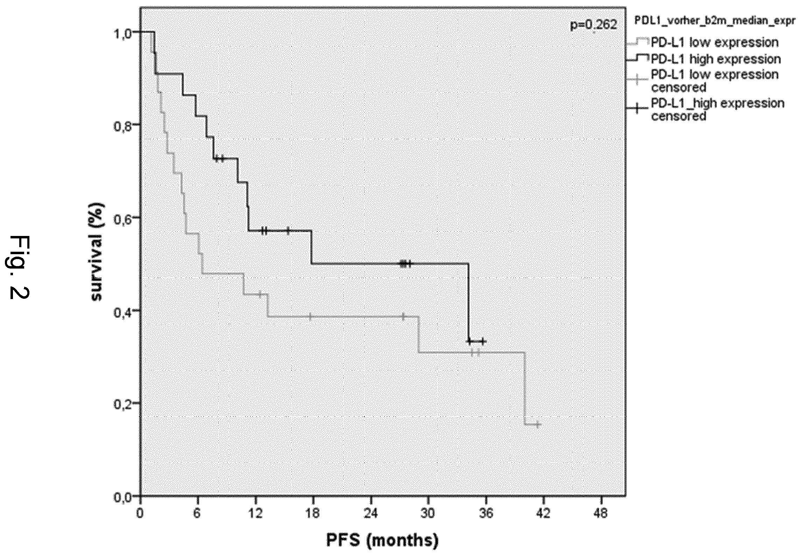

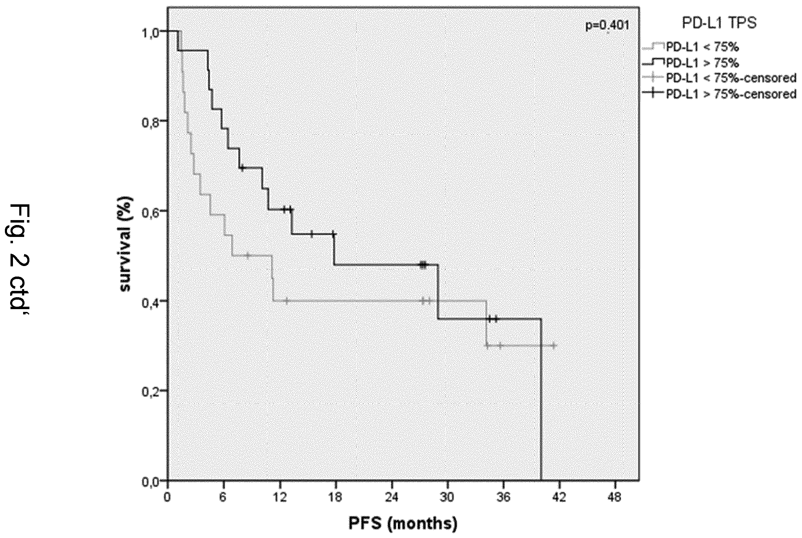

To identify peripheral blood biomarker candidates for pembrolizumab treatment, we examined five of those associated with the immune system (PD1, PD-L1, CTLA4, CD3, CD4, CD68, CD163 and CD8) before treatment initiation. Expressions divided by median in low and high expression revealed no significant difference for either PFS or OS in log rank test. Though not statistically significant, it was interesting to see, that PD-L1 TPS scores (<75% versus ≥75%) from the tumor tissue and PD-L1 expression (low versus high expression by median) from the LB revealed similar mPFS rates and Kaplan-Meier curves; the corresponding mPFS rates were 6.9 versus 17.8 months (TPS score p=0.401) and 6.4 and 17.8 months (gene expression p=0.262) for low and high expressers, respectively (Figure 2).

**Blood-based gene expression as ∆ before and after start of ICI treatment and survival**

Next, we performed measurements after start of ICI treatments of the five above mentioned biomarkers of the immune system. Pre- and post ICI mRNA expressions were comparable due to 40-∆ct values were all in the same range and therefore highly comparable. In addition, to evaluate a dynamic change, we categorized patients for every marker into decreased expression (∆ ≤ 0) and increased expression (∆ > 0) post treatment groups.

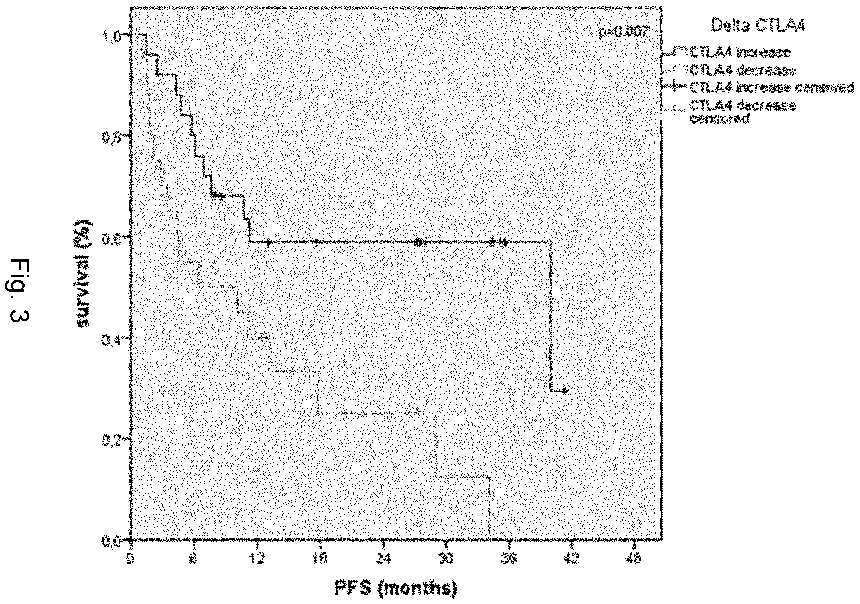

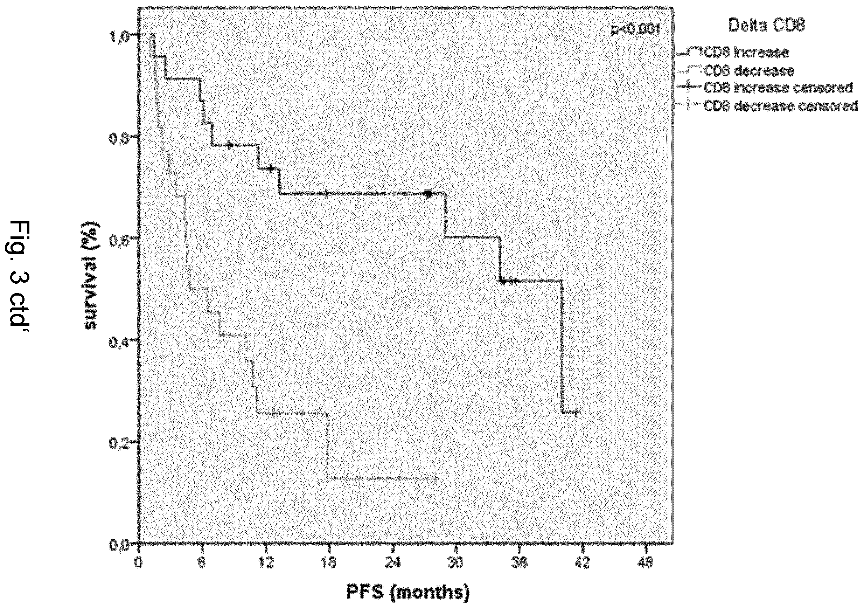

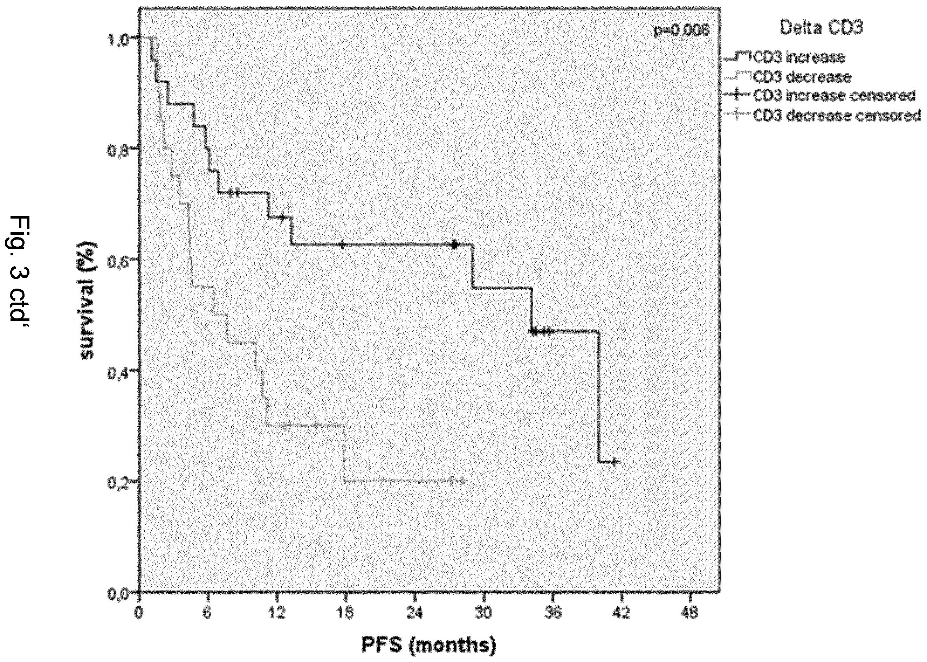

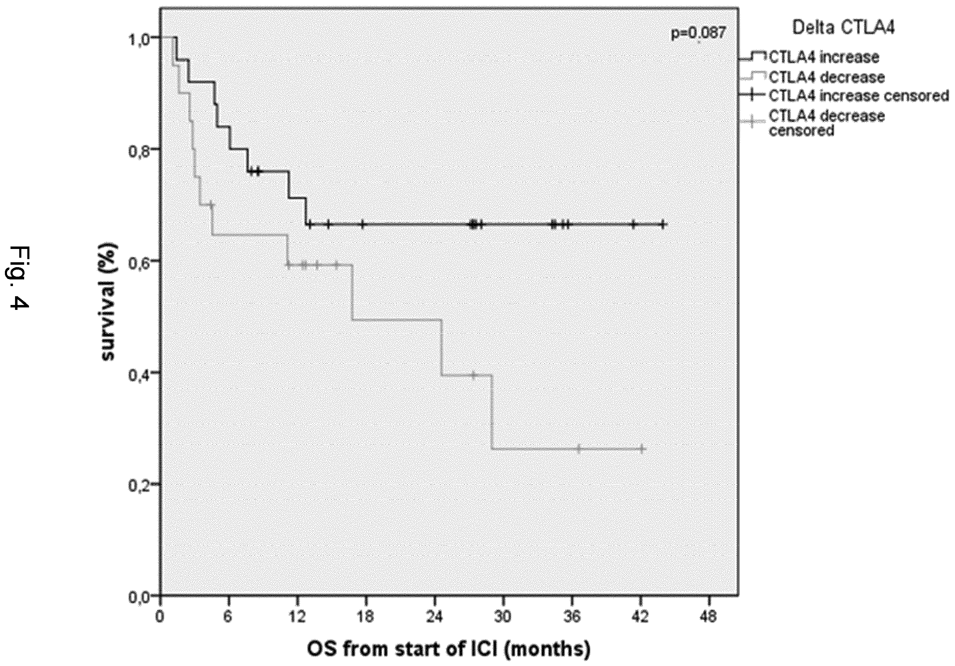

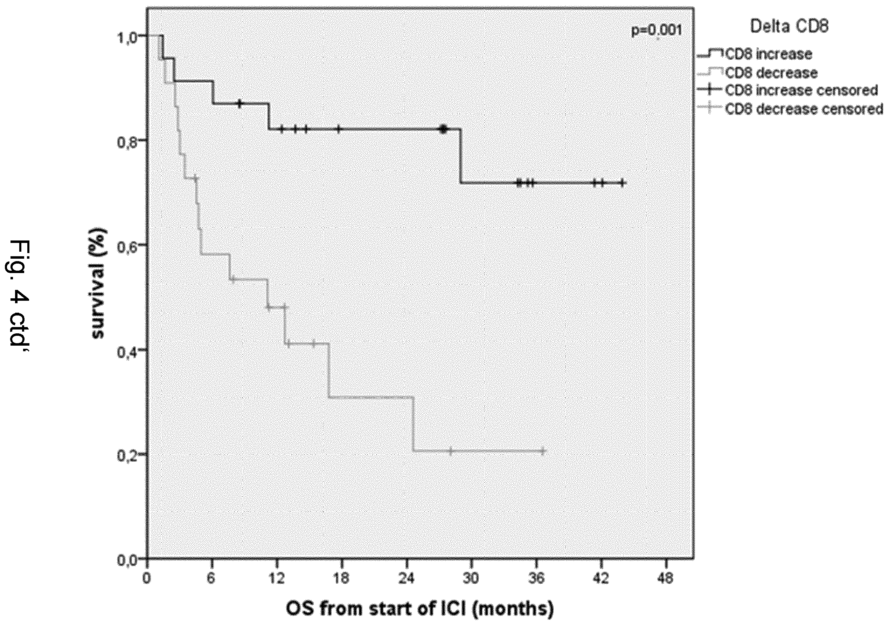

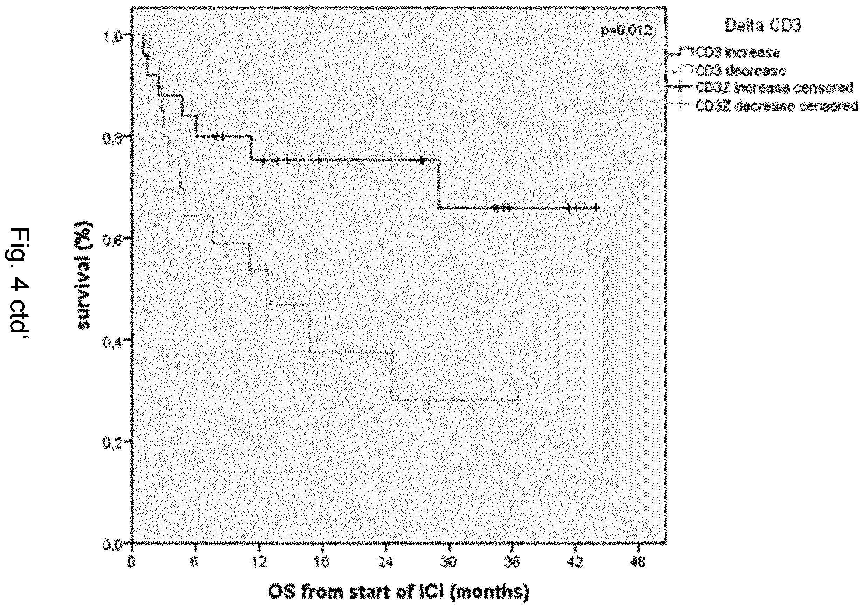

PFS was significantly improved by patients with increased blood plasma expression of CD3, CD8 and TCLA4 within 3 weeks after start of pembrolizumab therapy (Table 3). Especially, CD 8 was shown to be a good prognostic discriminator with mPFS rates of 40.0 months (increase) versus 4.7 months (decrease) (p< 0.001) (Figure 3). This prognostic value became true also for OS were CD8 showed again significant differences with mOS rates of n.r versus 11.1 months (p<0.001) Furthermore, CD 3 was of prognostic value in terms of OS with mOS rates of n.r. versus 12.7 months for increase versus decrease of this marker, respectively (Figure 4).

We performed univariate and multivariate analyses to test ∆ expressions for independent prognostic value. CD8 became the only independent prognostic marker for as well PFS as OS with p values of 0.011 and 0.006, respectively. (Tables 4 and 5).

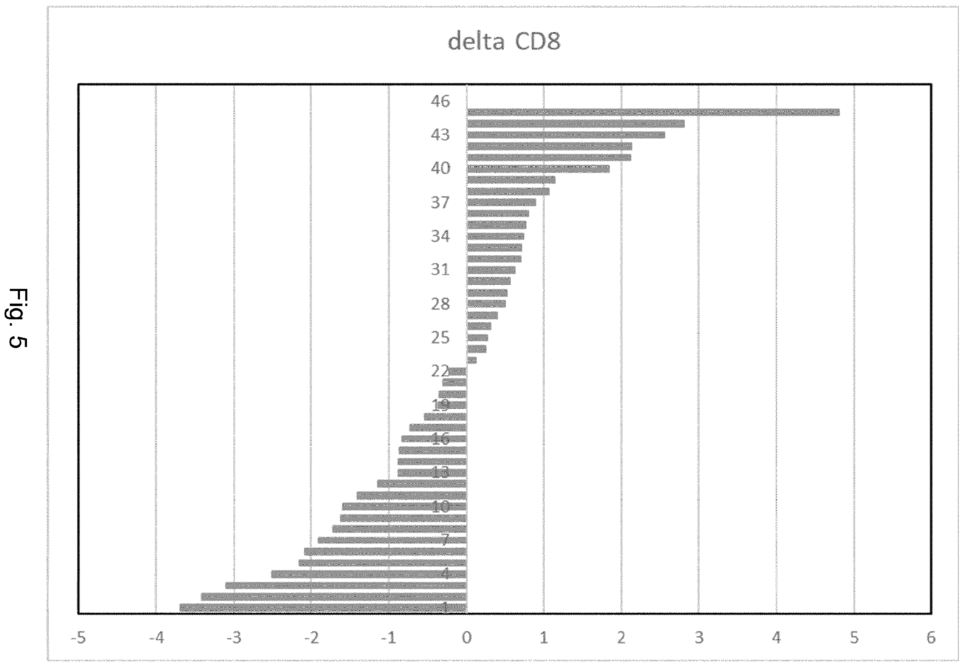

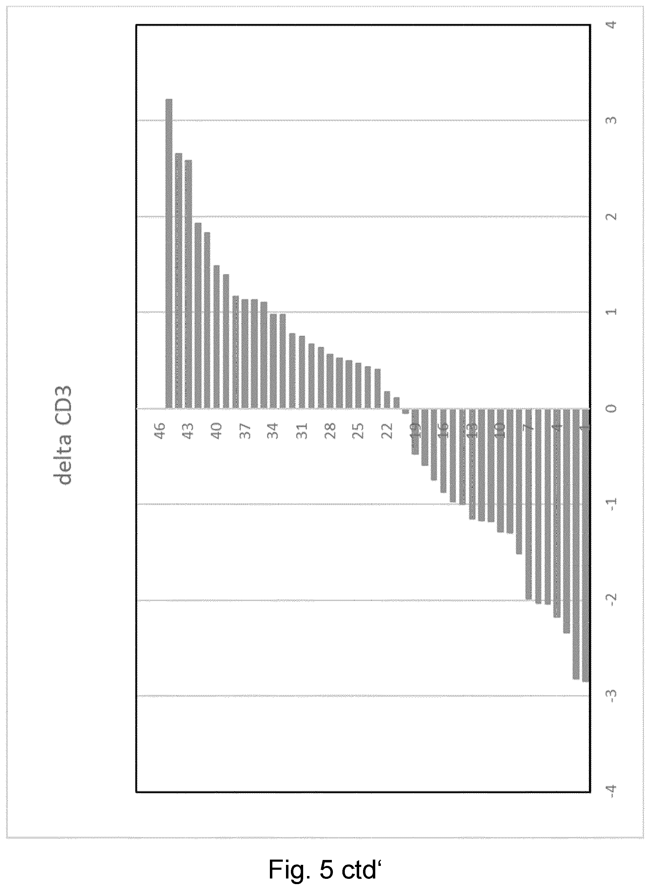

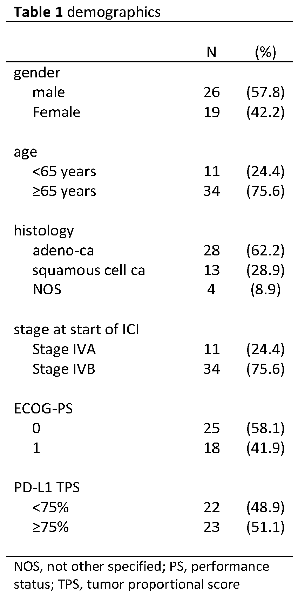

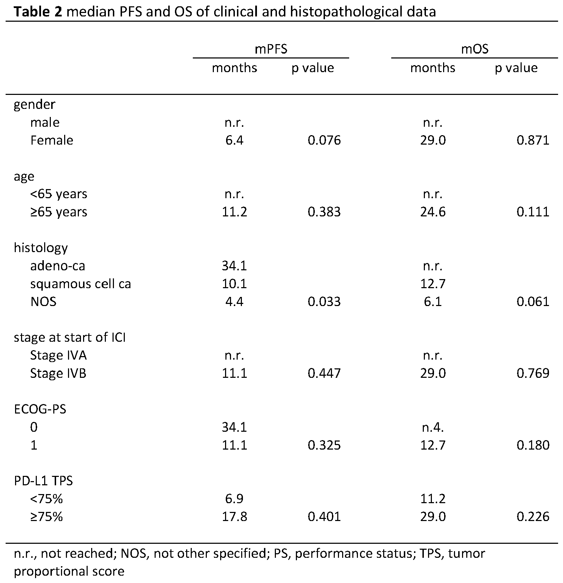

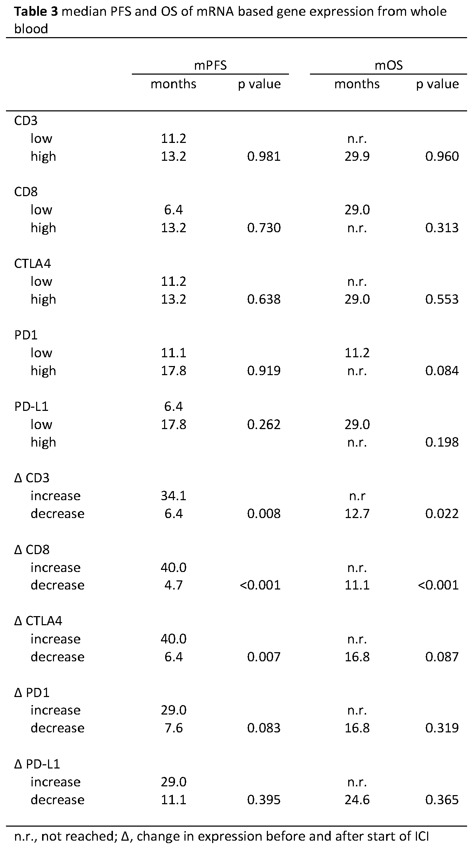

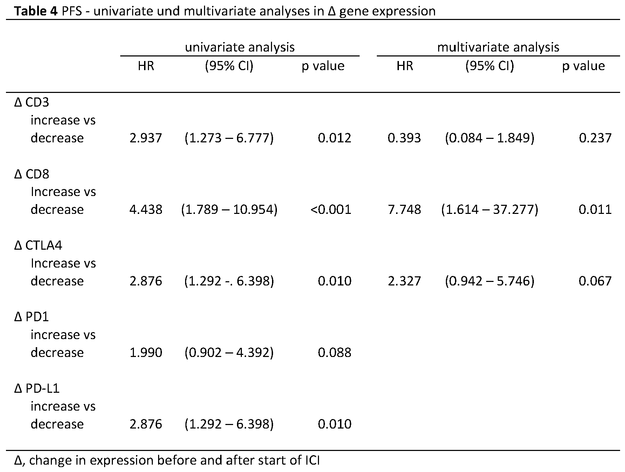

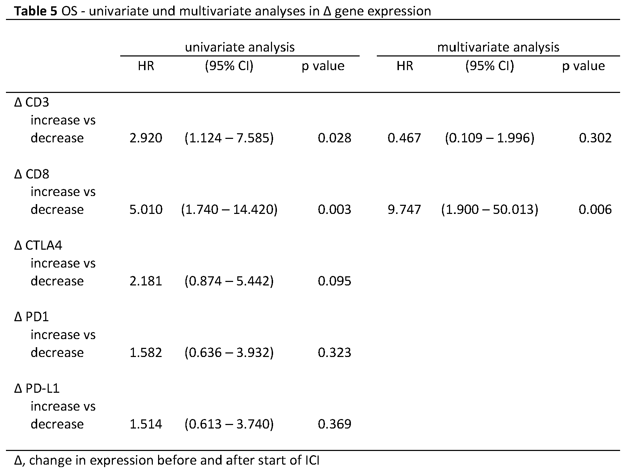

Changes in relative gene expression of blood cell markers before and after immune checkpoint therapy indicate, that almost half of the patients are allocated to be responders based on ∆ expressions >0 (Figure 5).

**References**

1. Borghaei H, Paz-Ares L, Horn L, Spigel DR, Steins M, Ready NE, et al. Nivolumab versus Docetaxel in Advanced Nonsquamous Non-Small-Cell Lung Cancer. N Engl J Med. 2015;373(17):1627-39.

2. Fehrenbacher L, Spira A, Ballinger M, Kowanetz M, Vansteenkiste J, Mazieres J, et al. Atezolizumab versus docetaxel for patients with previously treated non-small-cell lung cancer (POPLAR): a multicentre, open-label, phase 2 randomised controlled trial. Lancet. 2016;387(10030):1837-46.

3. Herbst RS, Baas P, Kim DW, Felip E, Perez-Gracia JL, Han JY, et al. Pembrolizumab versus docetaxel for previously treated, PD-L1-positive, advanced non-small-cell lung cancer (KEYNOTE-010): a randomised controlled trial. Lancet. 2016;387(10027): 1540-50.

4. Horn L, Spigel DR, Vokes EE, Holgado E, Ready N, Steins M, et al. Nivolumab Versus Docetaxel in Previously Treated Patients With Advanced Non-Small-Cell Lung Cancer: Two-Year Outcomes From Two Randomized, Open-Label, Phase III Trials (CheckMate 017 and CheckMate 057). J Clin Oncol. 2017;35(35):3924-33.

5. Rittmeyer A, Barlesi F, Waterkamp D, Park K, Ciardiello F, von Pawel J, et al. Atezolizumab versus docetaxel in patients with previously treated non-small-cell lung cancer (OAK): a phase 3, open-label, multicentre randomised controlled trial. Lancet. 2017;389(10066):255-65.

6. Gettinger S, Horn L, Jackman D, Spigel D, Antonia S, Hellmann M, et al. Five-Year Follow-Up of Nivolumab in Previously Treated Advanced Non-Small-Cell Lung Cancer: Results From the CA209-003 Study. J Clin Oncol. 2018;36(17): 1675-84.

7. Reck M, Rodriguez-Abreu D, Robinson AG, Hui R, Csoszi T, Fulop A, et al. Pembrolizumab versus Chemotherapy for PD-L1-Positive Non-Small-Cell Lung Cancer. N Engl J Med. 2016;375(19): 1823-33.

8. Reck M, Rodriguez-Abreu D, Robinson AG, Hui R, Csoszi T, Fulop A, et al. Five-Year Outcomes With Pembrolizumab Versus Chemotherapy for Metastatic Non-Small-Cell Lung Cancer With PD-L1 Tumor Proportion Score >/= 50. J Clin Oncol. 2021:JCO2100174.

9. Gandara D, Reck M, Moro-Sibilot D, Mazieres J, Gadgeel S, Morris S, et al. Fast progression in non-small cell lung cancer: results from the randomized phase III OAK study evaluating second-line atezolizumab versus docetaxel. J Immunother Cancer. 2021;9(3).

10. Brueckl WM, Ficker JH, Zeitler G. Clinically relevant prognostic and predictive markers for immune-checkpoint-inhibitor (ICI) therapy in non-small cell lung cancer (NSCLC). BMC Cancer. 2020;20(1):1185.

11. Rizvi NA, Hellmann MD, Snyder A, Kvistborg P, Makarov V, Havel JJ, et al. Cancer immunology. Mutational landscape determines sensitivity to PD-1 blockade in non-small cell lung cancer. Science. 2015;348(6230): 124-8.

12. Bodor JN, Boumber Y, Borghaei H. Biomarkers for immune checkpoint inhibition in non-small cell lung cancer (NSCLC). Cancer. 2020;126(2):260-70.

13. Diem S, Schmid S, Krapf M, Flatz L, Born D, Jochum W, et al. Neutrophil-to-Lymphocyte ratio (NLR) and Platelet-to-Lymphocyte ratio (PLR) as prognostic markers in patients with non-small cell lung cancer (NSCLC) treated with nivolumab. Lung Cancer. 2017;111:176-81.

14. Riedl JM, Barth DA, Brueckl WM, Zeitler G, Foris V, Mollnar S, et al. C-Reactive Protein (CRP) Levels in Immune Checkpoint Inhibitor Response and Progression in Advanced Non-Small Cell Lung Cancer: A Bi-Center Study. Cancers (Basel). 2020;12(8).

15. Chen S, Li R, Zhang Z, Huang Z, Cui P, Jia W, et al. Prognostic value of baseline and change in neutrophil-to-lymphocyte ratio for survival in advanced non-small cell lung cancer patients with poor performance status receiving PD-1 inhibitors. Transl Lung Cancer Res. 2021;10(3):1397-407.

16. Han J, Duan J, Bai H, Wang Y, Wan R, Wang X, et al. TCR Repertoire Diversity of Peripheral PD-1(+)CD8(+) T Cells Predicts Clinical Outcomes after Immunotherapy in Patients with Non-Small Cell Lung Cancer. Cancer Immunol Res. 2020;8(1):146-54.

17. Kamphorst AO, Pillai RN, Yang S, Nasti TH, Akondy RS, Wieland A, et al. Proliferation of PD-1+ CD8 T cells in peripheral blood after PD-1-targeted therapy in lung cancer patients. Proc Natl Acad Sci USA. 2017;114(19):4993-8.

18. Kim KH, Cho J, Ku BM, Koh J, Sun JM, Lee SH, et al. The First-week Proliferative Response of Peripheral Blood PD-1(+)CD8(+) T Cells Predicts the Response to Anti-PD-1 Therapy in Solid Tumors. Clin Cancer Res. 2019;25(7):2144-54.

19. Ricciuti B, Jones G, Severgnini M, Alessi JV, Recondo G, Lawrence M, et al. Early plasma circulating tumor DNA (ctDNA) changes predict response to first-line pembrolizumab-based therapy in non-small cell lung cancer (NSCLC). J Immunother Cancer. 2021;9(3).

20. Yamauchi T, Hoki T, Oba T, Jain V, Chen H, Attwood K, et al. T-cell CX3CR1 expression as a dynamic blood-based biomarker of response to immune checkpoint inhibitors. Nat Commun. 2021;12(1):1402.

21. Breyer J, Wirtz RM, Otto W, Erben P, Kriegmair MC, Stoehr R, et al. In stage pT1 non-muscle-invasive bladder cancer (NMIBC), high KRT20 and low KRT5 mRNA expression identify the luminal subtype and predict recurrence and survival. Virchows Arch. 2017;470(3):267-74.

22. Schmittgen TD, Livak KJ. Analyzing real-time PCR data by the comparative C(T) method. Nat Protoc. 2008;3(6): 1101-8.

23. Eisenhauer EA, Therasse P, Bogaerts J, Schwartz LH, Sargent D, Ford R, et al. New response evaluation criteria in solid tumours: revised RECIST guideline (version 1.1). Eur J Cancer. 2009;45(2):228-47.

24. Boussiotis VA. Molecular and Biochemical Aspects of the PD-1 Checkpoint Pathway. N Engl J Med. 2016;375(18):1767-78.

25. Tumeh PC, Harview CL, Yearley JH, Shintaku IP, Taylor EJ, Robert L, et al. PD-1 blockade induces responses by inhibiting adaptive immune resistance. Nature. 2014;515(7528):568-71.

26. Gros A, Parkhurst MR, Tran E, Pasetto A, Robbins PF, Ilyas S, et al. Prospective identification of neoantigen-specific lymphocytes in the peripheral blood of melanoma patients. Nat Med. 2016;22(4):433-8.

27. Eckstein M, Strissel P, Strick R, Weyerer V, Wirtz R, Pfannstiel C, et al. Cytotoxic T-cell-related gene expression signature predicts improved survival in muscle-invasive urothelial bladder cancer patients after radical cystectomy and adjuvant chemotherapy. J Immunother Cancer. 2020;8(1).

28. Riaz N, Havel JJ, Makarov V, Desrichard A, Urba WJ, Sims JS, et al. Tumor and Microenvironment Evolution during Immunotherapy with Nivolumab. Cell. 2017;171(4):934-49 e16.

29. Hopkins AC, Yarchoan M, Durham JN, Yusko EC, Rytlewski JA, Robins HS, et al. T cell receptor repertoire features associated with survival in immunotherapy-treated pancreatic ductal adenocarcinoma. JCI Insight. 2018;3(13).

30. Guibert N, Jones G, Beeler JF, Plagnol V, Morris C, Mourlanette J, et al. Targeted sequencing of plasma cell-free DNA to predict response to PD1 inhibitors in advanced non-small cell lung cancer. Lung Cancer. 2019;137:1-6.

31. Yang Q, Chen M, Gu J, Niu K, Zhao X, Zheng L, et al. Novel Biomarkers of Dynamic Blood PD-L1 Expression for Immune Checkpoint Inhibitors in Advanced Non-Small-Cell Lung Cancer Patients. Front Immunol. 2021;12:665133.

32. Gandhi L, Rodriguez-Abreu D, Gadgeel S, Esteban E, Felip E, De Angelis F, et al. Pembrolizumab plus Chemotherapy in Metastatic Non-Small-Cell Lung Cancer. N Engl J Med. 2018;378(22):2078-92.

33. Paz-Ares L, Luft A, Vicente D, Tafreshi A, Gumus M, Mazieres J, et al. Pembrolizumab plus Chemotherapy for Squamous Non-Small-Cell Lung Cancer. N Engl J Med. 2018;379(21):2040-51.

34. Paz-Ares L, Ciuleanu TE, Cobo M, Schenker M, Zurawski B, Menezes J, et al. First-line nivolumab plus ipilimumab combined with two cycles of chemotherapy in patients with non-small-cell lung cancer (CheckMate 9LA): an international, randomised, open-label, phase 3 trial. Lancet Oncol. 2021;22(2):198-211.

36. Kozera B, Rapacz M. Reference genes in real-time PCR. J Appl Genet. 2013 Nov;54(4):391-406. doi: 10.1007/s13353-013-0173-x. PMID: 24078518; PMCID: PMC3825189.

**Sequences**

The following sequences form part of the disclosure of the present application. A WIPO ST 25 compatible electronic sequence listing is provided with this application, too. For the avoidance of doubt, if discrepancies exist between the sequences in the following table and the electronic sequence listing, the sequences in this table shall be deemed to be the correct ones. The sequences are disclosed on groups of three, comprising a probe, a forward primer and a reverse primer each

**Table 7: Primer/Probe sets that can be used in the context of the present invention**

| SEQ ID No | Sequence | Qualifier |
|---|---|---|
| 1 | ACCCCTCAGCCGTGCCTGTGTTC | PD-1 probe |
| 2 | GGCCAGCCCCTGAAGGA | PD-1 forward primer |
| 3 | GGAAATCCAGCTCCCCATAGTC | PD-1 reverse primer |
| 4 | TGAGCCCCAGCAACCAGACGGA | PD-1 probe |
| 5 | CAACACATCGGAGAGCTTCGT | PD-1 forward primer |
| 6 | GGAAGGCGGCCAGCTT | PD-1 reverse primer |
| 7 | CAGAAGTGCCCTTTGCCTCCACTCAA | PD-L1 probe |
| 8 | CCCTAATTTGAGGGTCAGTTCCT | PD-L1 forward primer |
| 9 | CTCAGTCATGCAGAAAACAAATTGA | PD-L1 reverse primer |
| 10 | CAAAGTGATACACATTTGGAGGAGACGTAA | PD-L1 probe |
| 11 | TGGCATCCAAGATACAAACTCAA | PD-L1 forward primer |
| 12 | TTGAAGATCAGAAGTTCCAATGCT | PD-L1 reverse primer |
| 13 | CAAAGCTCTGTGCCTCTGTAATCGGCA | CD3 probe |
| 14 | CACCGCGGCCATCCT | CD3 forward primer |
| 15 | AGTTTGGGATCCAGCAGGC | CD3 reverse primer |
| 16 | ACATCAAGGTTCTGCCCACATGGTCCACCC | CD4 probe |
| 17 | GTGGCAGTGTCTGCTGAGTGA | CD4 forward primer |
| 18 | AGCACAATCAGGGCCATTG | CD4 reverse primer |
| 19 | TTCCTGCCAGCGAAGCCCAC | CD8 probe |
| 20 | TGAGCAACTCCATCATGTACTTCAG | CD8 forward primer |
| 21 | GGCGCCGGTGTTGGT | CD8 reverse primer |
| 22 | CAAATGTCCCCCGGCCTGTGG | CD8 probe |
| 23 | CACAGGAACCGAAGACGTGTT | CD8 forward primer |
| 24 | GGGCTTGTCTCCCGATTTG | CD8 reverse primer |
| 25 | TCCACCTCGACCTGCTCTCCCTGAG | CD68 probe |
| 26 | CAGCAACTCGAGCATCATTCTT | CD68 forward primer |
| 27 | GCAGGAGAAACTTTGCCCAAA | CD68 reverse primer |
| 28 | TGCTCAAAGGGAGCAGATCTGAGCCTGAGAC | CD163 probe |
| 29 | TGCTGAGGATGCTGGAGTGA | CD163 forward primer |

(continued)

| SEQ ID No | Sequence | Qualifier |
|---|---|---|
| 30 | GAACATTCAGTGACTCCATCTACCA | CD163 reverse primer |
| 31 | TCGCGTCTCGGAAACCGGTAGC | CALM2 probe |
| 32 | GAGCGAGCTGAGTGGTTGTG | CALM2 forward primer |
| 33 | AGTCAGTTGGTCAGCCATGCT | CALM2 reverse primer |
| 34 | ATGATGCTGCTTACATGTCTCGATCCCA | B2M probe |
| 35 | TGACTTTGTCACAGCCCAAGATA | B2M forward primer |
| 36 | AATGCGGCATCTTCAAACCT | B2M reverse primer |
| 37 | CTGGACGTACAATACCACTTCCGCTGTC | RPL37A probe |
| 38 | TGTGGTTCCTGCATGAAGACA | RPL37A forward primer |
| 39 | CAGTCTTCTGATGGCGGACTT | RPL37A reverse primer |
| 40 | CCCTGTCTTCTGCAAAGCAATGC | CTLA4 probe |
| 41 | GGCCCTGCACTCTCCTGTT | CTLA4 forward primer |
| 42 | AGTGGCTTTGCCTGGAGATG | CTLA4 reverse primer |

## Claims

1. A method for predicting a clinical response in a human or animal subject suffering from or at risk of developing a neoplastic disease towards at least one given mode of treatment, said method comprising the steps of:

   c) administering to the subject at least one initial dose of an immune checkpoint inhibitor
   d) after said initial administration, obtaining a biological sample from said subject
   e) determining, in said sample, the post-treatment expression level of at least one gene of interest selected from the group consisting of PD1, PD-L1, CTLA4, CD3, CD4, CD68, CD163 and CD8, and
   f) predicting therapeutic success for follow up treatment with

      i) at least the immune checkpoint inhibitor of step c) or
      ii) at least one other immune checkpoint inhibitor.

2. The method of claim 1, which method further comprises, before step c) the steps of

   a) obtaining a biological sample from said subject, and
   b) determining, in said sample, the pre-treatment expression level of at least one gene of interest selected from the group consisting of PD1, PD-L1, CTLA4, CD3, CD4, CD68, CD163 and CD8, and

3. The method according to claim 1 or 2, wherein in order to carry out step f), and after step e), it is determined whether the post-treatment expression level of the at least one gene of interest is high or low.

4. The method according to claim 3, wherein the post-treatment expression level of the at least one gene of interest is high

   (i) in case it is higher than a predetermined treshold for that respective gene of interest, or
   (ii) in case the post-treatment expression level determined in step e) is higher than or equal to the pre-treatment expression level in step b),

   whereas the post-treatment expression level of the at least one gene of interest is low

   (i) in case it is lower than a predetermined treshold for that respective gene of interest, or
   (ii) in case the post-treatment expression level determined in step e) is lower than the pre-treatment expression level in step b)

5. The method according to any one of claims 3 - 5 wherein

   • a high post-treatment expression level of at least one gene of interest is associated with good prognosis for treatment with at least one immune checkpoint inhibitor, and
   • a low post-treatment expression level of at least one gene of interest is associated with poor prognosis for treatment with at least one immune checkpoint inhibitor, and

6. The method according to any one of claims 1 - 6, wherein at least one gene of interest the expression of which is determined is a gene the gene product of which is a target of the immune checkpoint inhibitor of step f)(i) or f)(ii)

7. The method according to any one of the aforementioned claims, wherein the biological sample is at least one selected from the group consisting of

   • blood
   • plasma
   • serum
   • tissue
   • urine, and/or
   • saliva

8. The method according to any one of the aforementioned claims, wherein said expression level(s) is/are determined by real time polymerase chain reaction (RT-PCR or qPCR) of at least one of the genes of interest.
   preferably wherein the sample is treated with germanium beads or silica beads, or magnetic beads coated with germanium or silica, and a chaotropic salt, for purification of the nucleic acids contained in said sample prior to the determination in step e) and/or b)).

9. The method according to any one of the aforementioned claims, wherein the immune checkpoint inhibitor is at least one selected from the group consisting of

   • PD-1 inhibitor
   • PD-L1 inhibitor
   • CTLA-4 inhibitor
   • LAG 3, inhibitor
   • TIM3 inhibitor, and/or
   • OX40 inhibitor

   wherein the immune checkpoint inhibitor is at least one selected from the group as set forth in table 6.

10. An oligonucleotide comprising at least one nucleotide sequence which is capable of hybridizing to

    a) a nucleic acid molecule that encodes for any one of PD1, PD-L1, CTLA4, CD3, CD4, CD68, CD163 and CD8, or,
    b) an mRNA that encodes for any one of PD1, PD-L1, CTLA4, CD3, CD4, CD68, CD 163 and CD8,

    which oligonucleotide is selected from the group consisting of

    - an amplification primer (forward and/or reverse)
    - a labelled probe, and/or
    - a substrate bound probe

    for the manufacture of a kit for carrying out a method of any one of the aforementioned claims.

11. A kit comprising at least one oligonucleotide set forth in claim 10, which kit preferably comprises a set of forward/reverse primers capable of hybridizing to a nucleic acid molecule that encodes for at least one of PD1, PD-L1, CTLA4, CD3, CD4, CD68, CD163 and CD8, preferably at least one primer/probe set comprising a forward primer and a reverse primer, and optionally a probe, as set forth in table 7.

**12.** The kit according to claim 10, which comprises a labelled probe that is labelled with one or more fluorescent molecules, luminescent molecules, radioactive molecules, enzymatic molecules and/or quenching molecules.

**13.** An immune checkpoint inhibitor for use in the treatment of a human or animal subject

• being diagnosed for,
• suffering from, or
• being at risk of developing
a neoplastic disease,
wherein said subject or neoplastic disease has a high expression level in a given sample of at least one gene of interest selected from the group consisting of PD1, PD-L1, CTLA4, CD3 and CD 8, as determined after administration to said subject of at least one initial dose of an immune checkpoint inhibitor.

**14.** A method of treating a human or animal subject

• being diagnosed for,
• suffering from, or
• being at risk of developing
a neoplastic disease
which method comprising administering, in one or more therapeutically effective doses, an immune checkpoint inhibitor,
wherein said subject or neoplastic disease has a high expression level in a given sample of at least one gene of interest selected from the group consisting of PD1, PD-L1, CTLA4, CD3 and CD 8, as determined after administration to said subject of at least one initial dose of an immune checkpoint inhibitor.

Fig. 1

Fig. 1 ctd'

Fig. 2

Fig. 2 ctd'

Fig. 3

EP 4 112 746 A1

Fig. 3 ctd'

Fig. 3 ctd'

Fig. 4

Fig. 4 ctd'

Fig. 4 ctd'

Fig. 5

delta CD3

Fig. 5 ctd'

**Table 1** demographics

|  | N | (%) |
|---|---|---|
| gender |  |  |
| male | 26 | (57.8) |
| Female | 19 | (42.2) |
|  |  |  |
| age |  |  |
| <65 years | 11 | (24.4) |
| ≥65 years | 34 | (75.6) |
|  |  |  |
| histology |  |  |
| adeno-ca | 28 | (62.2) |
| squamous cell ca | 13 | (28.9) |
| NOS | 4 | (8.9) |
|  |  |  |
| stage at start of ICI |  |  |
| Stage IVA | 11 | (24.4) |
| Stage IVB | 34 | (75.6) |
|  |  |  |
| ECOG-PS |  |  |
| 0 | 25 | (58.1) |
| 1 | 18 | (41.9) |
|  |  |  |
| PD-L1 TPS |  |  |
| <75% | 22 | (48.9) |
| ≥75% | 23 | (51.1) |

NOS, not other specified; PS, performance status; TPS, tumor proportional score

**Table 2** median PFS and OS of clinical and histopathological data

| | mPFS | | mOS | |
| --- | --- | --- | --- | --- |
| | months | p value | months | p value |
| gender | | | | |
|   male | n.r. | | n.r. | |
|   Female | 6.4 | 0.076 | 29.0 | 0.871 |
| | | | | |
| age | | | | |
|   <65 years | n.r. | | n.r. | |
|   ≥65 years | 11.2 | 0.383 | 24.6 | 0.111 |
| | | | | |
| histology | | | | |
|   adeno-ca | 34.1 | | n.r. | |
|   squamous cell ca | 10.1 | | 12.7 | |
|   NOS | 4.4 | 0.033 | 6.1 | 0.061 |
| | | | | |
| stage at start of ICI | | | | |
|   Stage IVA | n.r. | | n.r. | |
|   Stage IVB | 11.1 | 0.447 | 29.0 | 0.769 |
| | | | | |
| ECOG-PS | | | | |
|   0 | 34.1 | | n.4. | |
|   1 | 11.1 | 0.325 | 12.7 | 0.180 |
| | | | | |
| PD-L1 TPS | | | | |
|   <75% | 6.9 | | 11.2 | |
|   ≥75% | 17.8 | 0.401 | 29.0 | 0.226 |

n.r., not reached; NOS, not other specified; PS, performance status; TPS, tumor proportional score

**Table 3** median PFS and OS of mRNA based gene expression from whole blood

| | mPFS | | mOS | |
|---|---|---|---|---|
| | months | p value | months | p value |
| **CD3** | | | | |
| low | 11.2 | | n.r. | |
| high | 13.2 | 0.981 | 29.9 | 0.960 |
| **CD8** | | | | |
| low | 6.4 | | 29.0 | |
| high | 13.2 | 0.730 | n.r. | 0.313 |
| **CTLA4** | | | | |
| low | 11.2 | | n.r. | |
| high | 13.2 | 0.638 | 29.0 | 0.553 |
| **PD1** | | | | |
| low | 11.1 | | 11.2 | |
| high | 17.8 | 0.919 | n.r. | 0.084 |
| **PD-L1** | 6.4 | | | |
| low | 17.8 | 0.262 | 29.0 | |
| high | | | n.r. | 0.198 |
| **Δ CD3** | | | | |
| increase | 34.1 | | n.r | |
| decrease | 6.4 | 0.008 | 12.7 | 0.022 |
| **Δ CD8** | | | | |
| increase | 40.0 | | n.r. | |
| decrease | 4.7 | <0.001 | 11.1 | <0.001 |
| **Δ CTLA4** | | | | |
| increase | 40.0 | | n.r. | |
| decrease | 6.4 | 0.007 | 16.8 | 0.087 |
| **Δ PD1** | | | | |
| increase | 29.0 | | n.r. | |
| decrease | 7.6 | 0.083 | 16.8 | 0.319 |
| **Δ PD-L1** | | | | |
| increase | 29.0 | | n.r. | |
| decrease | 11.1 | 0.395 | 24.6 | 0.365 |

n.r., not reached; Δ, change in expression before and after start of ICI

**Table 4** PFS - univariate und multivariate analyses in Δ gene expression

| | univariate analysis | | | multivariate analysis | | |
|---|---|---|---|---|---|---|
| | HR | (95% CI) | p value | HR | (95% CI) | p value |
| Δ CD3 increase vs decrease | 2.937 | (1.273 – 6.777) | 0.012 | 0.393 | (0.084 – 1.849) | 0.237 |
| Δ CD8 Increase vs decrease | 4.438 | (1.789 – 10.954) | <0.001 | 7.748 | (1.614 – 37.277) | 0.011 |
| Δ CTLA4 Increase vs decrease | 2.876 | (1.292 -. 6.398) | 0.010 | 2.327 | (0.942 – 5.746) | 0.067 |
| Δ PD1 increase vs decrease | 1.990 | (0.902 – 4.392) | 0.088 | | | |
| Δ PD-L1 increase vs decrease | 2.876 | (1.292 – 6.398) | 0.010 | | | |

Δ, change in expression before and after start of ICI

**Table 5** OS - univariate und multivariate analyses in Δ gene expression

| | univariate analysis | | | multivariate analysis | | |
|---|---|---|---|---|---|---|
| | HR | (95% CI) | p value | HR | (95% CI) | p value |
| Δ CD3 increase vs decrease | 2.920 | (1.124 − 7.585) | 0.028 | 0.467 | (0.109 − 1.996) | 0.302 |
| Δ CD8 increase vs decrease | 5.010 | (1.740 − 14.420) | 0.003 | 9.747 | (1.900 − 50.013) | 0.006 |
| Δ CTLA4 increase vs decrease | 2.181 | (0.874 − 5.442) | 0.095 | | | |
| Δ PD1 increase vs decrease | 1.582 | (0.636 − 3.932) | 0.323 | | | |
| Δ PD-L1 increase vs decrease | 1.514 | (0.613 − 3.740) | 0.369 | | | |

Δ, change in expression before and after start of ICI

Europäisches
Patentamt

European
Patent Office

Office européen
des brevets

# EUROPEAN SEARCH REPORT

Application Number

EP 21 18 3549

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/148790 A1 (AYERS MARK [US] ET AL) 31 May 2018 (2018-05-31) | 1-13 | INV. C12Q1/6886 |
| Y | * abstract; claims 1,5,12,13 * <br> * paragraph [0009]; tables 1A, 1B * <br> * RT-PCR; <br> paragraph [0099] * | 1-13 | |
| Y | WO 2020/172591 A1 (MITRA RXDX INC [US]) 27 August 2020 (2020-08-27) <br> * abstract; claims 1,9,13,16 * | 1-13 | |
| Y | US 2019/113513 A1 (SHAKED YUVAL [IL]) 18 April 2019 (2019-04-18) <br> * abstract; claims 1,13,22 * | 1-13 | |
| X | YANG QIAO ET AL: "Novel Biomarkers of Dynamic Blood PD-L1 Expression for Immune Checkpoint Inhibitors in Advanced Non-Small-Cell Lung Cancer Patients", FRONTIERS IN IMMUNOLOGY, vol. 12, 16 April 2021 (2021-04-16), XP055873945, DOI: 10.3389/fimmu.2021.665133 | 1-13 | |
| Y | * abstract * | 1-13 | |
| X | US 2021/009697 A1 (LEWIS KATHERINE E [US] ET AL) 14 January 2021 (2021-01-14) <br> * abstract; claim 135 * <br> * paragraphs [0025], [0029], [0212], [1063], [1066], [1201]; figures 11,13 * | 1-13 | |

TECHNICAL FIELDS SEARCHED (IPC)

C12Q

-/--

The present search report has been drawn up for all claims

| Place of search | Date of completion of the search | Examiner |
|---|---|---|
| The Hague | 21 March 2022 | Aguilera, Miguel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

**EUROPEAN SEARCH REPORT**

Application Number

EP 21 18 3549

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VILAIN RICARDO E. ET AL: "Dynamic Changes in PD-L1 Expression and Immune Infiltrates Early During Treatment Predict Response to PD-1 Blockade in Melanoma", CLINICAL CANCER RESEARCH, vol. 23, no. 17, 16 May 2017 (2017-05-16), pages 5024-5033, XP055873955, US ISSN: 1078-0432, DOI: 10.1158/1078-0432.CCR-16-0698 * abstract * * page 5025, column 1, paragraph 1 * * PD-1 marker + CD8 marker; page 5031, column 2, paragraph 2-3 * * CD8 marker; page 5030, column 1, paragraph 2; figure 1D * | 1-13 | |
| X | ZAPPASODI ROBERTA ET AL: "Strategies for Predicting Response to Checkpoint Inhibitors", CURRENT HEMATOLOGIC MALIGNANCY REPORTS, SPRINGER US, NEW YORK, vol. 13, no. 5, 29 August 2018 (2018-08-29), pages 383-395, XP036722597, ISSN: 1558-8211, DOI: 10.1007/S11899-018-0471-9 [retrieved on 2018-08-29] * abstract * * page 390, column 1, paragraph 1 * | 1-13 | TECHNICAL FIELDS SEARCHED (IPC) |

The present search report has been drawn up for all claims

| Place of search | Date of completion of the search | Examiner |
|---|---|---|
| The Hague | 21 March 2022 | Aguilera, Miguel |

Europäisches
Patentamt

European
Patent Office

Office européen
des brevets

**Application Number**

**EP 21 18 3549**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

**1-14(partially)**

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt

European Patent Office

Office européen des brevets

**LACK OF UNITY OF INVENTION**

**SHEET B**

Application Number

EP 21 18 3549

The Search Division considers that the present European patent application does not comply with the requirements of unity of invention and relates to several inventions or groups of inventions, namely:

```
1. claims: 1-14(partially)

    Methods for predicting clinical response to treatment with
    an immune checkpoint inhibitor comprising determining
    post-treatment expression level of PD-1. Oligonucleotides
    capable of hybridizing to a nucleic acid molecule that
    encodes PD-1. Kits comprising said oligonucleotides. Immune
    checkpoint inhibitor for use in the treatment and methods of
    treatment of subjects showing high expression levels of PD-1
    after administration of said inhibitor.


1.1. claims: 1-14(partially)

    Methods for predicting clinical response to treatment with
    an immune checkpoint inhibitor comprising determining
    post-treatment expression level of PD-L1. Oligonucleotides
    capable of hybridizing to a nucleic acid molecule that
    encodes PD-L1. Kits comprising said oligonucleotides. Immune
    checkpoint inhibitor for use in the treatment and methods of
    treatment of subjects showing high expression levels of
    PD-L1 after administration of said inhibitor.
                              ---


2-7. claims: 1-14(partially)

    Methods for predicting clinical response to treatment with
    an immune checkpoint inhibitor comprising determining
    post-treatment expression level of CTLA-4. Oligonucleotides
    capable of hybridizing to a nucleic acid molecule that
    encodes CTLA-4. Kits comprising said oligonucleotides.
    Immune checkpoint inhibitor for use in the treatment and
    methods of treatment of subjects showing high expression
    levels of CTLA-4 after administration of said inhibitor.
    .
    [idem for each one of the remaining expression markers
    listed in claim 1, i.e. CD3, CD4, CD68, CD163 and CD8]
                              ---


Please note that all inventions mentioned under item 1, although not
necessarily linked by a common inventive concept, could be searched
without effort justifying an additional fee.
```

## ANNEX TO THE EUROPEAN SEARCH REPORT
## ON EUROPEAN PATENT APPLICATION NO.

EP 21 18 3549

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2022

| Patent document cited in search report | | Publication date | Patent family member(s) | | Publication date |
|---|---|---|---|---|---|
| US 2018148790 | A1 | 31-05-2018 | EP | 3283882 A1 | 21-02-2018 |
| | | | EP | 3839510 A2 | 23-06-2021 |
| | | | ES | 2844799 T3 | 22-07-2021 |
| | | | US | 2018148790 A1 | 31-05-2018 |
| | | | WO | 2016168133 A1 | 20-10-2016 |
| WO 2020172591 | A1 | 27-08-2020 | WO | 2020172591 A1 | 27-08-2020 |
| | | | WO | 2020172592 A1 | 27-08-2020 |
| US 2019113513 | A1 | 18-04-2019 | AU | 2019396760 A1 | 01-07-2021 |
| | | | AU | 2019396761 A1 | 29-07-2021 |
| | | | CA | 3066053 A1 | 13-12-2018 |
| | | | CA | 3066054 A1 | 13-12-2018 |
| | | | CA | 3122732 A1 | 18-06-2020 |
| | | | CA | 3122738 A1 | 18-06-2020 |
| | | | CN | 113423467 A | 21-09-2021 |
| | | | CN | 113424062 A | 21-09-2021 |
| | | | EP | 3635403 A1 | 15-04-2020 |
| | | | EP | 3635410 A1 | 15-04-2020 |
| | | | EP | 3894013 A1 | 20-10-2021 |
| | | | EP | 3894860 A2 | 20-10-2021 |
| | | | JP | 2020522707 A | 30-07-2020 |
| | | | JP | 2020525758 A | 27-08-2020 |
| | | | JP | 2022512228 A | 02-02-2022 |
| | | | JP | 2022514480 A | 14-02-2022 |
| | | | US | 2019113513 A1 | 18-04-2019 |
| | | | US | 2019137495 A1 | 09-05-2019 |
| | | | WO | 2018225062 A1 | 13-12-2018 |
| | | | WO | 2018225063 A1 | 13-12-2018 |
| | | | WO | 2020121310 A2 | 18-06-2020 |
| | | | WO | 2020121315 A1 | 18-06-2020 |
| US 2021009697 | A1 | 14-01-2021 | AU | 2018334171 A1 | 05-03-2020 |
| | | | BR | 112020004879 A2 | 15-09-2020 |
| | | | CA | 3073531 A1 | 21-03-2019 |
| | | | CN | 111479586 A | 31-07-2020 |
| | | | EP | 3681535 A1 | 22-07-2020 |
| | | | JP | 2020535119 A | 03-12-2020 |
| | | | KR | 20200051024 A | 12-05-2020 |
| | | | SG | 11202001606X A | 30-03-2020 |
| | | | US | 2021009697 A1 | 14-01-2021 |
| | | | WO | 2019055537 A1 | 21-03-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0242759 A
- WO 0241992 A
- WO 02097413 A

**Non-patent literature cited in the description**

- **BORGHAEI H ; PAZ-ARES L ; HORN L ; SPIGEL DR ; STEINS M ; READY NE et al.** Nivolumab versus Docetaxel in Advanced Nonsquamous Non-Small-Cell Lung Cancer. *N Engl J Med,* 2015, vol. 373 (17), 1627-39
- **FEHRENBACHER L ; SPIRA A ; BALLINGER M ; KOWANETZ M ; VANSTEENKISTE J ; MAZIERES J et al.** Atezolizumab versus docetaxel for patients with previously treated non-small-cell lung cancer (POPLAR): a multicentre, open-label, phase 2 randomised controlled trial. *Lancet,* 2016, vol. 387 (10030), 1837-46
- **HERBST RS ; BAAS P ; KIM DW ; FELIP E ; PEREZ-GRACIA JL ; HAN JY et al.** Pembrolizumab versus docetaxel for previously treated, PD-L1-positive, advanced non-small-cell lung cancer (KEYNOTE-010): a randomised controlled trial. *Lancet,* 2016, vol. 387 (10027), 1540-50
- **HORN L ; SPIGEL DR ; VOKES EE ; HOLGADO E ; READY N ; STEINS M et al.** Nivolumab Versus Docetaxel in Previously Treated Patients With Advanced Non-Small-Cell Lung Cancer: Two-Year Outcomes From Two Randomized, Open-Label, Phase III Trials (CheckMate 017 and CheckMate 057). *J Clin Oncol,* 2017, vol. 35 (35), 3924-33
- **RITTMEYER A ; BARLESI F ; WATERKAMP D ; PARK K ; CIARDIELLO F ; VON PAWEL J et al.** Atezolizumab versus docetaxel in patients with previously treated non-small-cell lung cancer (OAK): a phase 3, open-label, multicentre randomised controlled trial. *Lancet,* 2017, vol. 389 (10066), 255-65
- **GETTINGER S ; HORN L ; JACKMAN D ; SPIGEL D ; ANTONIA S ; HELLMANN M et al.** Five-Year Follow-Up of Nivolumab in Previously Treated Advanced Non-Small-Cell Lung Cancer: Results From the CA209-003 Study. *J Clin Oncol,* 2018, vol. 36 (17), 1675-84
- **RECK M ; RODRIGUEZ-ABREU D ; ROBINSON AG ; HUI R ; CSOSZI T ; FULOP A et al.** Pembrolizumab versus Chemotherapy for PD-L1-Positive Non-Small-Cell Lung Cancer. *N Engl J Med,* 2016, vol. 375 (19), 1823-33
- **RECK M ; RODRIGUEZ-ABREU D ; ROBINSON AG ; HUI R ; CSOSZI T ; FULOP A et al.** Five-Year Outcomes With Pembrolizumab Versus Chemotherapy for Metastatic Non-Small-Cell Lung Cancer With PD-L1 Tumor Proportion Score >/= 50. *J Clin Oncol,* 2021
- **GANDARA D ; RECK M ; MORO-SIBILOT D ; MAZIERES J ; GADGEEL S ; MORRIS S et al.** Fast progression in non-small cell lung cancer: results from the randomized phase III OAK study evaluating second-line atezolizumab versus docetaxel. *J Immunother Cancer,* 2021, vol. 9 (3
- **BRUECKL WM ; FICKER JH ; ZEITLER G.** Clinically relevant prognostic and predictive markers for immune-checkpoint-inhibitor (ICI) therapy in non-small cell lung cancer (NSCLC). *BMC Cancer,* 2020, vol. 20 (1), 1185
- **RIZVI NA ; HELLMANN MD ; SNYDER A ; KVISTBORG P ; MAKAROV V ; HAVEL JJ et al.** Cancer immunology. Mutational landscape determines sensitivity to PD-1 blockade in non-small cell lung cancer. *Science,* 2015, vol. 348 (6230), 124-8
- **BODOR JN ; BOUMBER Y ; BORGHAEI H.** Biomarkers for immune checkpoint inhibition in non-small cell lung cancer (NSCLC). *Cancer,* 2020, vol. 126 (2), 260-70
- **DIEM S ; SCHMID S ; KRAPF M ; FLATZ L ; BORN D ; JOCHUM W et al.** Neutrophil-to-Lymphocyte ratio (NLR) and Platelet-to-Lymphocyte ratio (PLR) as prognostic markers in patients with non-small cell lung cancer (NSCLC) treated with nivolumab. *Lung Cancer,* 2017, vol. 111, 176-81
- **RIEDL JM ; BARTH DA ; BRUECKL WM ; ZEITLER G ; FORIS V ; MOLLNAR S et al.** C-Reactive Protein (CRP) Levels in Immune Checkpoint Inhibitor Response and Progression in Advanced Non-Small Cell Lung Cancer: A Bi-Center Study. *Cancers (Basel),* 2020, vol. 12 (8

- **CHEN S ; LI R ; ZHANG Z ; HUANG Z ; CUI P ; JIA W et al.** Prognostic value of baseline and change in neutrophil-to-lymphocyte ratio for survival in advanced non-small cell lung cancer patients with poor performance status receiving PD-1 inhibitors. *Transl Lung Cancer Res,* 2021, vol. 10 (3), 1397-407
- **HAN J ; DUAN J ; BAI H ; WANG Y ; WAN R ; WANG X et al.** TCR Repertoire Diversity of Peripheral PD-1(+)CD8(+) T Cells Predicts Clinical Outcomes after Immunotherapy in Patients with Non-Small Cell Lung Cancer. *Cancer Immunol Res,* 2020, vol. 8 (1), 146-54
- **KAMPHORST AO ; PILLAI RN ; YANG S ; NASTI TH ; AKONDY RS ; WIELAND A et al.** Proliferation of PD-1+ CD8 T cells in peripheral blood after PD-1-targeted therapy in lung cancer patients. *Proc Natl Acad Sci USA.,* 2017, vol. 114 (19), 4993-8
- **KIM KH ; CHO J ; KU BM ; KOH J ; SUN JM ; LEE SH et al.** The First-week Proliferative Response of Peripheral Blood PD-1(+)CD8(+) T Cells Predicts the Response to Anti-PD-1 Therapy in Solid Tumors. *Clin Cancer Res,* 2019, vol. 25 (7), 2144-54
- **RICCIUTI B ; JONES G ; SEVERGNINI M ; ALESSI JV ; RECONDO G ; LAWRENCE M et al.** Early plasma circulating tumor DNA (ctDNA) changes predict response to first-line pembrolizumab-based therapy in non-small cell lung cancer (NSCLC). *J Immunother Cancer,* 2021, vol. 9 (3
- **YAMAUCHI T ; HOKI T ; OBA T ; JAIN V ; CHEN H ; ATTWOOD K et al.** T-cell CX3CR1 expression as a dynamic blood-based biomarker of response to immune checkpoint inhibitors. *Nat Commun,* 2021, vol. 12 (1), 1402
- **BREYER J ; WIRTZ RM ; OTTO W ; ERBEN P ; KRIEGMAIR MC ; STOEHR R et al.** In stage pT1 non-muscle-invasive bladder cancer (NMIBC), high KRT20 and low KRT5 mRNA expression identify the luminal subtype and predict recurrence and survival. *Virchows Arch,* 2017, vol. 470 (3), 267-74
- **SCHMITTGEN TD ; LIVAK KJ.** Analyzing real-time PCR data by the comparative C(T) method. *Nat Protoc,* 2008, vol. 3 (6), 1101-8
- **EISENHAUER EA ; THERASSE P ; BOGAERTS J ; SCHWARTZ LH ; SARGENT D ; FORD R et al.** New response evaluation criteria in solid tumours: revised RECIST guideline (version 1.1). *Eur J Cancer,* 2009, vol. 45 (2), 228-47
- **BOUSSIOTIS VA.** Molecular and Biochemical Aspects of the PD-1 Checkpoint Pathway. *N Engl J Med,* 2016, vol. 375 (18), 1767-78
- **TUMEH PC ; HARVIEW CL ; YEARLEY JH ; SHINTAKU IP ; TAYLOR EJ ; ROBERT L et al.** PD-1 blockade induces responses by inhibiting adaptive immune resistance. *Nature,* 2014, vol. 515 (7528), 568-71
- **GROS A ; PARKHURST MR ; TRAN E ; PASETTO A ; ROBBINS PF ; ILYAS S et al.** Prospective identification of neoantigen-specific lymphocytes in the peripheral blood of melanoma patients. *Nat Med,* 2016, vol. 22 (4), 433-8
- **ECKSTEIN M ; STRISSEL P ; STRICK R ; WEYERER V ; WIRTZ R ; PFANNSTIEL C et al.** Cytotoxic T-cell-related gene expression signature predicts improved survival in muscle-invasive urothelial bladder cancer patients after radical cystectomy and adjuvant chemotherapy. *J Immunother Cancer,* 2020, vol. 8 (1
- **RIAZ N ; HAVEL JJ ; MAKAROV V ; DESRICHARD A ; URBA WJ ; SIMS JS et al.** Tumor and Microenvironment Evolution during Immunotherapy with Nivolumab. *Cell,* 2017, vol. 171 (4), 934-49e16
- **HOPKINS AC ; YARCHOAN M ; DURHAM JN ; YUSKO EC ; RYTLEWSKI JA ; ROBINS HS et al.** T cell receptor repertoire features associated with survival in immunotherapy-treated pancreatic ductal adenocarcinoma. *JCI Insight,* 2018, vol. 3 (13
- **GUIBERT N ; JONES G ; BEELER JF ; PLAGNOL V ; MORRIS C ; MOURLANETTE J et al.** Targeted sequencing of plasma cell-free DNA to predict response to PD1 inhibitors in advanced non-small cell lung cancer. *Lung Cancer,* 2019, vol. 137, 1-6
- **YANG Q ; CHEN M ; GU J ; NIU K ; ZHAO X ; ZHENG L et al.** Novel Biomarkers of Dynamic Blood PD-L1 Expression for Immune Checkpoint Inhibitors in Advanced Non-Small-Cell Lung Cancer Patients. *Front Immunol,* 2021, vol. 12, 665133
- **GANDHI L ; RODRIGUEZ-ABREU D ; GADGEEL S ; ESTEBAN E ; FELIP E ; DE ANGELIS F et al.** Pembrolizumab plus Chemotherapy in Metastatic Non-Small-Cell Lung Cancer. *N Engl J Med,* 2018, vol. 378 (22), 2078-92
- **PAZ-ARES L ; LUFT A ; VICENTE D ; TAFRESHI A ; GUMUS M ; MAZIERES J et al.** Pembrolizumab plus Chemotherapy for Squamous Non-Small-Cell Lung Cancer. *N Engl J Med,* 2018, vol. 379 (21), 2040-51
- **PAZ-ARES L ; CIULEANU TE ; COBO M ; SCHENKER M ; ZURAWSKI B ; MENEZES J et al.** First-line nivolumab plus ipilimumab combined with two cycles of chemotherapy in patients with non-small-cell lung cancer (CheckMate 9LA): an international, randomised, open-label, phase 3 trial. *Lancet Oncol,* 2021, vol. 22 (2), 198-211
- **KOZERA B ; RAPACZ M.** Reference genes in real-time PCR. *J Appl Genet,* November 2013, vol. 54 (4), 391-406